(19) Europäisches Patentamt
European Patent Office
Office européen des brevets

(11) **EP 2 040 607 B1**

(12) **EUROPÄISCHE PATENTSCHRIFT**

(45) Veröffentlichungstag und Bekanntmachung des Hinweises auf die Patenterteilung:
**09.09.2015  Patentblatt 2015/37**

(21) Anmeldenummer: **07787243.0**

(22) Anmeldetag: **09.07.2007**

(51) Int Cl.:
***A61B 3/15*** *(2006.01)*

(86) Internationale Anmeldenummer:
**PCT/EP2007/056967**

(87) Internationale Veröffentlichungsnummer:
**WO 2008/003788 (10.01.2008 Gazette 2008/02)**

(54) **OPHTHALMOSKOP**

OPHTHALMOSCOPE

OPHTALMOSCOPE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **07.07.2006  EP 06116853**

(43) Veröffentlichungstag der Anmeldung:
**01.04.2009  Patentblatt 2009/14**

(60) Teilanmeldung:
**11075183.1 / 2 386 243**
**11075184.9 / 2 386 244**

(73) Patentinhaber: **OD-OS GmbH**
**14513 Teltow (DE)**

(72) Erfinder:
• **LIESFELD, Ben**
**14469 Potsdam (DE)**
• **TEIWES, Winfried**
**14532 Kleinmachnow (DE)**
• **HUPPERTZ, Michael**
**14513 Teltow (DE)**
• **AMTHOR, Kay-Uwe**
**14469 Potsdam (DE)**

(74) Vertreter: **Pfenning, Meinig & Partner GbR**
**Patent- und Rechtsanwälte**
**Joachimsthaler Strasse 12**
**10719 Berlin (DE)**

(56) Entgegenhaltungen:
**WO-A-02/094088     US-B2- 6 758 564**

EP 2 040 607 B1

## Beschreibung

Die vorliegende Erfindung betrifft ein Ophthalmoskop zur Untersuchung eines Auges eines Patienten.

Derartige optische Geräte werden insbesondere zur Beobachtung des Augenhintergrunds des Patienten eingesetzt, beispielsweise für Netzhautuntersuchungen. Ein Ophthalmoskop muß hochauflösende Farb- bzw. Schwarzweißbilder in kontinuierlicher Abfolge liefern, um zur Diagnose des Auges ebenso wie bei der Durchführung und Dokumentation von therapeutischen Eingriffen eingesetzt werden zu können. Hierbei stellt die Abbildung des Augenhintergrunds, beispielsweise der Netzhaut, eine optische Herausforderung dar, denn sowohl die Beleuchtung als auch die Beobachtung des Augenhintergrunds müssen durch eine verhältnismäßig kleine Eintrittspupille des Auges durchgeführt werden. Zudem weist der Augenhintergrund in der Regel nur eine schwache Reflektivität auf, die für rote Farbanteile dominiert, so dass kontrastreiche Farbbilder vom Augenhintergrund in der Regel nur mit einer Lichtquelle mit starken Blau- und Grünanteilen erzeugt werden können.

Neben der Erzeugung von einfachen Farbbildern der Netzhaut werden Ophthalmoskope auch für komplexere Bildgebungsverfahren verwendet wie z. B. für den qualitativen Nachweis der Fluoreszenz eines Farbstoffes, der in die Blutbahn des Patienten eingebracht wurde, in den Gefäßen der Netzhaut. Es ist daher die Aufgabe der vorliegenden Erfindung, derartige Fluoreszenzmessungen zu verbessern. Diese Aufgabe wird erfindungsgemäß gelöst durch ein Ophthalmoskop gemäß dem Patentanspruch 1.

Die optische Untersuchung des Augenhintergrunds mit Hilfe eines Beleuchtungsstrahls, der nach Reflexion beispielsweise an der Netzhaut als Beobachtungsstrahl untersucht wird, wird zudem durch unerwünschte weitere Reflexionen an den Grenzflächen der Kornea sowie durch unerwünschte Lichtstreuung beispielsweise an Glaskörpertrübungen erschwert. Im nachfolgenden werden all diese störenden Lichtstrahlen, die im Beobachtungsstrahl enthalten sein können, jedoch nicht auf die gewünschte Reflexion des Beleuchtungsstrahls am zu untersuchenden Teil des Auges zurückzuführen sind, zusammenfassend als "Streulicht" bezeichnet.

Zur Lösung dieses Streulichtproblems offenbart die EP 1389943 B1 ein herkömmliches Ophthalmoskop zur Untersuchung eines Auges eines Patienten, umfassend:

eine Beleuchtungsvorrichtung zur Erzeugung eines Beleuchtungsstrahls;
eine Beleuchfungs-Abbildungsoptik zum Abbilden des Beleuchtungsstrahls auf das Auge, und
Mittel zum Scannen des Beleuchtungsstrahls über das Auge, sowie
eine Beobachtungsvorrichtung, die einen elektronischen Sensor mit einem Feld von fotosensitiven Pixeln umfaßt, die jeweils zeilenweise aktivierbar und/oder auslesbar sind;
eine Beobachtungs-Abbildungsoptik zum Abbilden eines durch Reflexion des Beleuchtungsstrahls am Auge erzeugten Beobachtungsstrahls auf die Beobachtungsvorrichtung, und
Mittel zum Ausblenden von Streulicht aus dem Beobachtungsstrahl.

Als Beleuchtungsvorrichtung wird hierbei insbesondere eine Halogenlampe eingesetzt, deren Licht mittels eines Kondensors kollimiert und über die Beleuchtungs-Abbildungsoptik auf den Augenhintergrund des Patienten fokussiert wird. Der am Augenhintergrund reflektierte Beobachtungsstrahl wird über die Beobachtungs-Abbildungsoptik auf eine Bildebene abgebildet, in der sich ein CCD-Sensor als Beobachtungsvorrichtung befindet.

Die Mittel zum Scannen des Beleuchtungsstrahls über das Auge sind bei diesem herkömmlichen Ophthalmoskop durch eine Schlitzblende gebildet, die vor der Halogenlampe in dem vom Kondensor kollimierten Beleuchtungsstrahl oszilliert. Diese Blende ist aus einem lichtundurchlässigen Material, beispielsweise einem Flachblechmaterial, gefertigt und läßt nur einen durch die Größe der Schlitzblende definierten linienförmigen Ausschnitt aus dem Beleuchtungsstrahl durch, der aufgrund der Oszillation der Schlitzblende somit ebenfalls mit dieser Oszillationsfrequenz über das Auge des Patienten hin- und her gescannt wird.

Auch die Mittel zum Ausblenden von Streulicht aus dem Beobachtungsstrahl sind bei diesem herkömmlichen Ophthalmoskop durch eine mechanisch oszillierende Schlitzblende gebildet. Insbesondere schlägt die EP 1389943 B1 vor, die vor der Halogenlampe oszillierende Schlitzblende und die vor dem CCD-Sensor oszillierende Schlitzblende, die aus Abbildungsgründen ohnehin miteinander synchronisiert werden müssen, als Blendenspaltpaar in einem gemeinsamen Flachblech auszubilden.

In der Praxis hat sich hierbei gezeigt, dass insbesondere die Wahl eines vor dem Sensor hin- und her schwingenden Blechs mit einer Schlitzblende zu Problemen führt. Denn einerseits lassen sich die Schwingungen des mechanischen Oszillators für das Blech nur ungenügend vom Gehäuse des Gerätes entkoppeln, so dass allgemein Schwierigkeiten in der Handhabung des herkömmlichen Ophthalmoskops und insbesondere Beeinträchtigungen in der Bildschärfe aufgrund eines Mitvibrierens des Sensors auftreten.

Andererseits sollte zur Erzielung einer möglichst kompakten Ausführung des Ophthalmoskops die Blende im Beobachtungsstrahl möglichst exakt in der Bildebene der Beobachtungsvorrichtung und somit auf dem Sensor liegen. Es muß jedoch ein gewisser Mindestabstand des mechanisch hin- und her schwingenden Blechs vom Sensor eingehalten werden, was unweigerlich zu einer schlechteren Unterdrückung störenden Streulichts führt,

Es ist daher nützlich, ein herkömmliches Ophthalmoskop derart weiterzuentwickeln, dass die Zahl mechanisch schwingender Bauteile verringert wird, insbesondere in der Nähe der Beobachtungsvorrichtung.

Zu diesem Zweck wird hier vorgeschlagen, dass die Mittel zum Ausblenden von Streulicht aus dem Beobachtungsstrahl eine elektronische Steuerschaltung zum Auslesen wenigstens einer Pixelzeile des Sensors umfassen, und dass das Ophthalmoskop eine elektronische Kontrolleinheit umfaßt, die dazu ausgelegt ist, die Mittel zum Scannen und die elektronische Steuerschaltung derart zu kontrollieren, dass das Scannen des Beleuchtungsstrahls über das Auge synchron mit der Änderung der aktuell auszulesenden Pixelzeile erfolgt.

Derartige elektronische Sensoren, insbesondere CMOS-Sensoren, sind im Stand der Technik an sich bekannt, vgl, beispielsweise die WO 99/05853. Der Einsatz eines derartigen elektronischen Sensors bei einem Ophthalmoskop erlaubt es nun erstmals, die im Stand der Technik benutzte mechanische Blende gleichsam durch eine elektronische Blende ("rolling shutter") zu ersetzen. Denn mit Hilfe der elektronischen Steuerschaltung können alle Pixelzeilen eines derartigen Sensors gezielt aktiviert, deaktiviert und ausgelesen werden. Im aktivierten Zustand eines Pixels führt Lichteinfall zur kontinuierlichen Erzeugung von Ladungen, die bei einem CMOS-Sensor über eine dem jeweiligen Pixel zugeordnete Verstärkerelektronik in ein Spannungssignal umgesetzt werden. Im deaktivierten Zustand hingegen unterbleibt ein derartiger Spannungsaufbau, das Pixel oder auch die gesamte Pixelzeile ist gleichsam "abgeschaltet".

Durch gezieltes Aktivieren wenigstens einer Pixelzeile in einem bestimmten Bereich des Felds von fotosensitiven Pixeln und gleichzeitiges Deaktivieren aller übrigen Pixelzeilen kann somit auf elektronische Weise das gleiche Ergebnis wie mit Hilfe einer mechanischen Schlitzblende erzielt werden. Beim hier vorgeschlagenen Ophthalmoskop kann somit Streulicht aus dem Beobachtungsstrahl ausgeblendet werden, ohne dass hierfür ein mechanisch schwingendes Blech mit einer Schlitzblende vor der Beobachtungsvorrichtung erforderlich ist. Zudem befindet sich die hier vorgeschlagene elektronische Blende im Gegensatz zur mechanischen Blende beim herkömmlichen Ophthalmoskop nicht vor, sondern in der Bildebene der Beobachtungsvorrichtung, was die Streulichtunterdrückung verbessert.

Zweckmäßigerweise ist vorgesehen, dass die elektronische Steuerschaltung dazu ausgelegt ist, jeweils eine einzelne Pixelzeile auszulesen. Auf diese Weise läßt sich eine äußerst feine Auflösung des elektronischen Sensors erzielen, die bei typischen CMOS-Sensoren einer Höhe von ca. 5μm entspricht. Es wäre jedoch grundsätzlich auch vorstellbar, mit Hilfe der elektronischen Steuerschaltung mehrere Pixelzeilen, insbesondere benachbarte Pixelzeilen, zusammenzufassen, d.h. stets gemeinsam zu aktivieren, zu deaktivieren oder auszulesen, und somit unter Verschlechterung der Auflösung die Lichtausbeute zu verbessern.

Zweckmäßigerweise ist die elektronische Steuerschaltung dazu ausgelegt, jeweils nach Ablauf einer einstellbaren Zeitverzögerung (Δt) die aktuell auszulesende Pixelzeile zu ändern, insbesondere von einer Pixelzeile zu einer benachbarten Pixelzeile. In der bevorzugten Ausführungsvariante, bei der unmittelbar benachbarte Pixelzeilen nacheinander ausgelesen werden, benötigt die elektronische Steuerschaltung somit für einen vollständigen Durchlauf, d.h. ein vollständiges Auslesen des elektronischen Sensors mit N Pixelzeilen, insgesamt eine Zeitdauer T=NxΔt. Hierbei kann der Paramter N, der die Zahl der Pixelzeilen. des Sensors beschreibt, ausgehend von einer durch die Bauart des Sensors vorgegebenen Obergrenze durch das oben beschriebene elektronische Zusammenfassen von benachbarten Pixelzeilen reduziert werden, während gleichzeitig der Zeitverzögerungsparameter Δt vom Bediener des hier vorgeschlagenen Ophthalmoskops frei einstellbar ist. Auf diese Weise kann die für einen vollständigen Auslese-Durchlauf benötigte Gesamtzeitdauer eingestellt werden, entsprechend der Einstellung der Schwingungsperiodendauer bzw. der Schwingungsfrequenz der beim herkömmlichen Ophthalmoskop vorgesehenen mechanischen Schlitzblende.

In einer bevorzugten Ausführungsform der Erfindung ist die elektronische Steuerschaltung ferner dazu ausgelegt, jede Pixelzeile während einer einstellbaren Belichtungszeit ($t_{INT}$) vor ihrem Auslesen zu aktivieren. Durch Einstellen dieser Belichtungszeit $t_{INT}$ als Vielfaches der Zeitverzögerung Δt läßt sich somit erreichen, dass jede Pixelzeile während eines längeren Zeitraums $t_{INT}$ belichtet wird, während dessen sich ihr die aktuell auszulesende Pixelzeile nähert. Auf diese Weise kann die effektive Breite der elektronischen Spaltblende flexibel eingestellt werden.

Zweckmäßigerweise ist die elektronische Steuerschaltung dazu ausgelegt, den Auslesevorgang infolge eines externen Triggersignals zu starten. Auf diese Weise kann die auch beim erfindungsgemäßen Ophthalmoskop erforderliche Synchronisierung der Blende für die Beobachtungsvorrichtung mit den Mitteln zum Scannen des Beleuchtungsstrahls sichergestellt werden. Beispielsweise kann ein zentraler Steuercomputer für das erfindungsgemäße Ophthalmoskop einerseits mit Hilfe dieses Triggersignals die elektronische Steuerschaltung des elektronischen Sensors und andererseits einen Antrieb für eine mechanische Schlitzblende im Beleuchtungsstrahl kontrollieren.

Grundsätzlich läßt sich mit Hilfe eines derartigen zentralen Steuercomputers eine Synchronisierung der elektronischen Blende im Beobachtungsstrahl mit einer mechanischen Blende im Beleuchtungsstrahl problemlos erzielen. Der zentrale Steuercomputer kann selbstverständlich auch Teil des elektronischen Sensors oder Teil der Beleuchtungsvorrichtung sein.

Um nun jedoch auch im Beleuchtungsstrahl eine mechanisch schwingende Schlitzblende überflüssig zu machen, kann in einer vorteilhaften Weiterbildung des erfindungsgemäßen Ophthalmoskops vorgesehen sein, dass die Mittel zum Scannen des Beleuchtungsstrahls über das Auge einen schwenkbar gelagerten Spiegel mit einer Scanvorrichtung umfassen. Um sicherzustellen, dass der auf den schwenkbar gelagerten Spiegel auftreffende Beleuchtungsstrahl bereits die gewünschte Linienform besitzt, kann beim erfindungsgemäßen Ophthalmoskop entweder vorgesehen sein, dass es ferner eine feste Spaltblende zum Auskoppeln eines linienförmigen Beleuchtungsstrahls umfaßt, oder es ferner eine Linienfokussieroptik zum Fokussieren des Beleuchtungsstrahls in einer Linie umfaßt, wobei in diesem letztgenannten Fall zweckmäßigerweise die Linienfokussieroptik eine Zylinderlinse umfaßt. Die Verwendung eines derartigen schwenkbar gelagerten Spiegels, der mit Hilfe eines Galvanometerantriebs angetrieben und bei einem Ophthalmoskop eingesetzt wird, um einen von einer Linienfokussieroptik gelieferten Beleuchtungsstrahl zu scannen, ist aus der US 6,758,564 B2 grundsätzlich bekannt, auf die insofern verwiesen wird.

Für eine zentrale Kontrolle der wesentlichen Bauteile des erfindungsgemäßen Ophthalmoskops ist zweckmäßigerweise vorgesehen, dass die elektronische Kontrolleinheit dazu ausgelegt ist, die elektronische Steuerschaltung des elektronischen Sensors und die Scanvorrichtung des schwenkbaren Spiegels zu kontrollieren. Die elektronische Kontrolleinheit kann dann durch Senden des oben besprochenen Triggersignals an die elektronische Steuerschaltung des elektronischen Sensors sicherstellen, dass bei einem beispielsweise sinusförmig schwingenden Spiegel der Auslesevorgang im elektronischen Sensor zu einem geeigneten Zeitpunkt startet, beispielsweise durch ein Auslesen der oberen Pixelzeile des elektronischen Sensors, sobald der schwingende Spiegel seinen oberen Umkehrpunkt erreicht hat.

Bei dieser Weiterbildung des erfindungsgemäßen Ophthalmoskops ist die elektronische Kontrolleinheit dazu ausgelegt, die Scanvorrichtung und die elektronische Steuerschaltung derart zu kontrollieren, dass das Scannen des Beleuchtungsstrahls über das Auge synchron mit der Änderung der aktuell auszulesenden Pixelzeile erfolgt. Beispielsweise kann die elektronische Kontrolleinheit der Scanvorrichtung eine Sägezahntrajektorie vorgeben, die exakt der Bewegung des elektronischen Spalts im elektronischen Sensor entspricht.

Für besonders präzise Untersuchungen des Auges des Patienten mit besonders hoher Ortsauflösung ist vorgesehen, dass die Stärke der Linie des fokussierten Beleuchtungsstrahls der Höhe einer Pixelzeile des elektronischen Sensors entspricht. Man erzielt hierdurch eine konfokale Abbildung des Auges, wodurch sich dreidimensionale Informationen über das beobachtete Objekt gewinnen lassen.

Bei Verwendung der besprochenen Linienfokussieroptik kann die Beleuchtungsvorrichtung zweckmäßigerweise ein Laser sein. Alternativ kann jede andere Form einer im wesentlichen punktförmigen Beleuchtungsvorrichtung eingesetzt werden, beispielsweise ein Ende eines beleuchteten Lichtwellenleiters.

In einer Weiterbildung der Erfindung ist vorgesehen, dass die Beobachtungsvorrichtung zwei Sensoren zur stereoskopischen Untersuchung des Auges umfaßt. Bei einer derartigen Ausführungsform werden die Vorteile der vorliegenden Erfindung besonders deutlich, da für eine stereoskopische Betrachtung mit zwei Beobachtungsvorrichtungen im Stand der Technik insgesamt sogar drei hin- und her oszillierende Schlitzblenden erforderlich sind, auf die erfindungsgemäß weitgehend bzw. bei Verwendung der Linienfokussieroptik sogar vollständig verzichtet werden kann.

Ferner ist in einer weiteren Ausführungsform der Erfindung vorgesehen, dass sie eine separate Lichtquelle und einen separaten Bildsensor zur Untersuchung des Auges umfaßt. Insbesondere kann hierbei mit Hilfe der Beleuchtungsvorrichtung, der Beleuchtungs-Abbildungsoptik, der Beobachtungs-Abbildungsoptik und der Beobachtungsvorrichtung der Augenhintergrund untersucht werden, beispielsweise die Netzhaut des Auges, während die zusätzliche Abbildungseinheit zur Untersuchung des Augenvordergrunds dient. Dies kann sowohl der medizinischen Untersuchung des Augenvordergrunds selbst dienen, als auch der automatisierten Positionierung des Auges des Patienten, als auch einer Verfolgung von Augenbewegungen (Tracking) des gesamten Auges anhand der Informationen, die man beispielsweise durch Reflexion eines Lichtstrahls am Augenvordergrund erhält. Insbesondere beim Aufsummieren von Augenhintergrund-Bildern bei Angiographie ist es sinnvoll, mittels einer derartigen gleichzeitigen Beobachtung des Augenvordergrunds Augenbewegungen feststellen zu können, die sich zwischen den einzelnen Bildern des Augenhintergrunds ereignet haben.

Vorteilhafterweise läßt sich das erfindungsgemäße Ophthalmoskop um Therapiefunktionen erweitern, wie nachfolgend erläutert werden wird:

Eine wichtige Therapieform in der Augenheilkunde zur Behandlung von Krankheiten wie Diabetischer Retinopathie oder altersbedingter Makuladegeneration ist die thermische Einwirkung durch Licht auf zu behandelnde Gewebebereiche, wie z. B. die photodynamische Therapie (PDT), die Photokoagulation von Gewebe oder neuere Verfahren wie selektive Retina-Therapie (SRT) und Therapie unterhalb der Koagulationsschwelle (Sub-Threshold Therapy, STT). Bei allen diesen Therapie-Formen wird ein intensiver Lichtimpuls gebündelt auf ein spezielles Gebiet der Netzhaut geleitet. Bei der Anwendung der Therapie ist darauf zu achten, dass wichtige Gebiete des Augenhintergrundes (z. B. die Makula) nicht versehentlich geschädigt werden.

Der behandelnde Arzt benötigt daher bei dieser Art des therapeutischen Eingriffs neben einem Live-Bild vom Augenhintergrund eine sehr präzise Kontrolle über die Position des Therapie-Lichtstrahls. Als Hilfsmittel zur Positionierung des Therapie-Lichstrahls wird typischerweise ein Zielstrahl dem Therapie-Lichtstrahl koaxial überlagert. Dieser ist für den Arzt immer sichtbar, besizt aber eine andere Wellenlänge und eine weit geringere Intensität als der Therapie-Lichtstrahl und führt daher nicht zu Veränderungen des zu Augenhintergrundes. Reflexe von den verschiedenen Grenzflächen des Auges, die durch die Lichtquelle zur Beleuchtung des Augenhintergrundes wie auch durch den Zielstrahl erzeugt werden, erschweren die Kontrolle jedoch erheblich.

Ein Ophthalmoskop, das ein reflexfreies Live-Bild des Augenhintergrundes erzeugt und gleichzeitig einen Therapiestrahl, dem i. d. R. ein Zielstrahl anderer Wellenlänge überlagert ist, auf den Augenhintergrund projiziert, stellt ein wichtiges Instrument für die Augenheilkunde dar. Es ermöglicht die Unterstützung des Arztes durch den Computer z. B. bei der Navigation über den Augenhintergrund zur Positionierung der Photokoagulationspunkte. Eine solche Computer-Unterstützung ist in anderen Bereichen der Medizin (bspw. der minimalinvasiven Chirurgie oder der Neurochirurgie) bereits Stand der Technik. In der Augenheilkunde scheitert ihre Implementierung bisher an den mangelhaften Beobachtungs- und Behandlungsinstrumenten. Diese technische Lücke kann die vorliegende Erfindung schließen.

Durch die Computer-Unterstützung kann ausserdem der therapeutische Eingriff objektiv dokumentiert werden. Bisher ist dies nur näherungsweise möglich, indem z. B. bei der Photokoagulation die Netzhaut durch den Lichtimpuls derart stark erhitzt wird, dass eine dauerhafte und deutlich sichtbare Veränderung des Gewebes entsteht. Dies führt dazu, dass die zerstörten Gewebeareale und damit der Verlust an Sehkraft nach der Therapie, meist viel größer sind als medizinisch notwendig. Moderne Behandlungsverfahren wie PDT, SRT und STT zielen gerade darauf, sichtbare Schädigungen der Netzhaut zu vermeiden. Ohne die mit der vorliegenden Erfindung mögliche Dokumentationsverfahren ist eine Qualitätssicherung bei diesen Therapien nicht möglich.

Typischerweise wird für die Behandlung der Netzhaut mit intensiven Lichtpulsen eine Spaltlampe verwendet, auf die ein Photokoagulationslaser mit einer entsprechenden Einkopplungsoptik adaptiert ist. Eine Spaltlampe ist ein Stereo-Mikroskop mit einer variablen Spaltbeleuchtung, wobei das Mikroskop wie auch die Beleuchtung um eine gemeinsame Achse in einer Ebene drehbar gelagert sind.

Zur Beobachtung des Augenhintergrundes hält der Benutzer mit der Hand eine ophthalmoskopische Linse oder ein Kontaktglas vor bzw. auf das Auge des Patienten. Das Therapielasersystem besteht meist aus der koaxialen Kombination aus Zielstrahl und Therapiestrahl, die zweckmäßigerweise, um ihre Unterscheidung zu erleichtern, verschiedene Wellenlängen haben. Der Therapiestrahl wird i. d. R. zwischen Mikroskop und ophthalmoskopischer Linse koaxial zum Beobachtungsstrahlengang eingekoppelt.

Es ist daher unvermeidbar, dass insbesondere der Zielstrahl auf den verschiedenen Grenzflächen zwischen Spaltlampe und Augenhintergrund starke Reflexe erzeugt, die vom Beobachter als störend wahrgenommen werden. Diese Reflexe sind sehr viel heller als das vom Augenhintergrund reflektierte Licht und können vom Betrachter nur mühsam durch geschicktes Bewegen/Ausrichten von Mikroskop und ophthalmoskopischer Linse oder Kontaktglas an den Rand des Bildfeldes gebracht werden, bzw. sie müssen einfach vom Betrachter toleriert werden. Reflexionen an Grenzschichten des menschlichen Auges sind grundsätzlich technisch (d. h. durch Vergütung der Oberflächen) nicht vermeidbar.

Die genannten Reflexe des Zielstrahls behindern jedoch nicht nur die Arbeit des Benutzers, der durch Okulare des Mikroskops schaut, sondern erschweren oder verhindern auch ggf. den Einsatz von Systemen, die Bilder des Augenhintergrundes mit Hilfe elektronischer Sensoren aufnehmen und den Benutzer während der Therapie durch computergestützte Auswertung unterstützen sollen.

Die vorliegende Erfindung beseitigt die bestehenden Einschränkungen/Nachteile des Standes der Technik, indem sie in einer weiteren Ausführungsform eine Vorrichtung zur Projektion eines Ziel- und Therapiestrahls bietet und die reflexfreie Beobachtung des Augenhintergrundes mit dem auf ihn projizierten Zielstrahl ermöglicht. Es wird ein Zielstrahl eingesetzt, der nicht polarisiert sein muss, was die Kosten für die Lichtquelle und die verwendete Optik reduziert. Reflexe, die durch den Zielstrahl hervorgerufen werden könnten, werden vollständig unterdrückt. Dabei ist nur ein einziger Detektor zur Verfolgung des Zielstrahls auf dem Objekt nötig.

In der vorliegenden Erfindung wird der Therapiestrahl mit dem ihm überlagerten Zielstrahl mit Hilfe eines Strahlteilers in den Strahlengang des erfindungsgemäßen Instruments eingekoppelt. Dies kann auf vielfältige Art und Weise erfolgen und ist z. T. in der EP 1389943 B1 (Abb. 3-5) offenbart. Der Therapiestrahl kann dabei koaxial zum Beleuchtungsstrahlengang oder koaxial zur Hauptinstrumentenachse angeordnet sein. Er darf jedoch nicht koaxial zum Beobachtungsstrahlengang liegen, da dies dem angewandten Prinzip zur Reflexunterdrückung widerspricht, das auf einer Trennung der Strahlengänge in der Pupillenebene des Auges beruht. Die Trennung der Strahlengänge führt dazu, dass das Bild eines durch den Zielstrahl an einer optischen Grenzschicht zwischen Sensor- und Objektebene hervorgerufenen Reflexes nicht an der gleichen Position auf dem Sensor entsteht wie das Bild des auf dem Objekt (hier: der Augenhintergrund) erzeugten Zielstrahlflecks.

Der Strahlteiler kann zweckmäßigerweise hochreflektiv für die Wellenlänge des Therapiestrahls, teilweise jedoch durchlässig für die Wellenlänge des Zielstrahls ausgeprägt sein. Dadurch erreicht nur Licht des Zielstrahls und nicht des Therapiestrahls den Detektor.

Alternativ kann der Strahlteiler wellenlängenunabhängig ausgeführt sein und im Beobachtungsstrahlengang ein zusätzlicher selektiver Filter eingesetzt werden, der das Licht des Therapiestrahls unterdrückt. Unerwünschte Reflexe und Streulicht, die durch den Zielstrahl verursacht werden, werden erfindungsgemäß dadurch unterdrückt, dass der Zielstrahl in einer Weise gepulst betrieben wird, die im Folgenden beschrieben wird.

Betrachtet man einen festen Punkt auf dem Objekt, auf den der Zielstrahl gerichtet ist, wird der Zielstrahl erfindungsgemäß nur zu genau dem Zeitpunkt $T_2$ für die Zeitdauer $T_p$ eingeschaltet, wenn die Beobachtungsblende des konfokalen Abbildungssystems genau diesen Objektpunkt überstreicht. Die Synchronisation der Aktivierung des Zielstrahls mit der konfokalen Blendenanordnung wird erfindungsgemäß durch eine Steuereinheit hergestellt. Diese Steuereinheit verfügt über Informationen über die jeweilige Position des Zielstrahlflecks auf dem Objekt und über den Startzeitpunkt der Blendenbewegung. Vorzugsweise ist diese Steuereinheit mit der Steuereinheit, die die Synchronisation von Beleuchtungs- und Beobachtungsblendenbewegung herstellt, identisch.

Dies kann beispielsweise dadurch realisiert sein, dass die Steuereinheit ein analoges oder digitales Positionssignal der Steuerspiegel des Zielstrahls sowie einen Synchronisationsimpuls der Blendenbewegung empfängt. Die Position des Zielstrahlflecks auf dem Objekt kann z. B. über die Position eines oder mehrerer Umlenkspiegel, die die Winkelauslenkung des Zielstrahls bestimmen, a priori bekannt sein. Aus der Position des Zielstrahlflecks, (x, y), und der Geschwindigkeit der Blendenbewegung, v, läßt sich eine zeitliche Verzögerung $\tau=y/v$ (bei Bewegung der Blende in $\gamma$-Richtung) zwischen dem Startzeitpunkt der Blendenbewegung und der Aktivierung des Zielstrahls berechnen und in eine entsprechende zweckmäßigerweise rechteckförmige Pulsfolge umsetzen. Wird der Zielstrahl auf dem Objekt bewegt, wird die zeitliche Verzögerung entsprechend der Position angepaßt.

Die erfindungsgemäße Vorrichtung stellt minimale Anforderungen an die verwendete Therapie- und Zielstrahlquelle. Die verwendeten Quellen müssen nicht notwendigerweise polarisiert sein und der Pulsbetrieb, der erfindungsgemäß auf einer Zeitskala von $100\mu s...10ms$ stattfindet, ist an sich bekannt.

In einer weiteren vorteilhaften Ausführungsform der Erfindung wird eine Steuereinheit eingesetzt, die die durch das erfindungsgemäße Instrument mit dem Sensor aufgenommenen Bilder insbesondere in Echtzeit auswertet. Diese Steuereinheit führt eine oder mehrere der folgenden Funktionen aus:

- Erkennung bzw. Bestimmung eines auf dem Objekt ortsfesten, daher objectbezogenen Koordinatensystems anhand von Strukturen auf dem Objekt in den von der erfindungsgemäßen Vorrichtung aufgenommenen Bildern bzw. auf Bildern desselben Objektes, die mit anderen Vorrichtungen aufgenommen wurden,

- Bestimmung der Position des Zielstrahls in diesem objektbezogenen Koordinatensystem,

- Positionierung des Zielstrahlflecks auf dem Augenhintergrund nach Vorgabe des Benutzers über eine geeignete Schnittstelle (z. B. einen Joystick) durch einen oder mehrere geeignete durch die Steuereinheit veränderliche Umlenkspiegel,

- Positionierung des Zielstrahlflecks ohne direkte Einwirkung des Benutzers durch einen oder mehrere geeignete durch die Steuereinheit veränderliche Umlenkspiegel,

- Freigabe bzw. Unterbindung der Auslösung eines Therapie-Lichtimpulses,

- Steuerung der Parameter des Therapie-Lichtimpulses (wie z. B. im Falle der Photokoagulation der Pulsdauer, Pulswiederholrate, Lichtleistung etc.),

- Messung der Dauer, Intensität und Häufigkeit der TherapieLichtimpulse,

- Speicherung der Steuerungsparameter der Therapie-Lichtimpulse und Speicherung der gemessenen Werte.

Durch Ausführung dieser Funktionen sind im Zusammenspiel mit einer geeigneten Anzeige (z. B. einem Bildschirm) folgende Verfahren möglich:

- Der Benutzer definiert Bereiche (Zonen) im objektbezogenen Koordinatensystem auf dem Objekt, in denen die Applikation von Therapie-Lichtimpulsen durch die Steuereinheit generell zugelassen oder generell unterbunden wird. Dies kann anhand eines mit dem erfindungsgemäßen Gerät zuvor erfassten Einzelbildes oder auch unter Verwendung von Bildern anderer Geräte nach dem Stand der Technik erfolgen. Die Steuereinheit bestimmt zu diesem Zweck in Echtzeit die Position des Zielstrahls im objektgebundenen Koordinatensystem und vergleicht die Position mit den vorgegebenen Bereichen und erteilt ggf. eine Freigabe für bzw. unterbindet die Auslösung eines Therapie-Lichtimpulses. In einer weiteren Ausführungsform führt die Steuereinheit die Definition der o. g. Zonen durch analyse des Bildes automatisch aus.

- Die Steuereinheit überlagert Informationen auf die Anzeige des Live-Bildes, wobei die Transformationen der Position der überlagerten Informationen in Bezug auf das Live-Bild kompensiert werden, d. h. sowohl der Position der überlagerten Informationen wie auch dem Live-Bild liegt das gleiche Objekt-bezogene Koordinatensystem zugrunde. Die Überlagerung kann durch schnellen Bildwechsel oder durch kontrastbasiertes Überblenden erfolgen. Die Informationen können z. B. aus Bildern des Objektes, die zu einem früheren Zeitpunkt oder unter Verwendung anderer Bildgebungsverfahren mit einem anderen Instrument aufgenommen wurden, oder vom Benutzer zu einem anderen Zeitpunkt erstellte graphische Darstellungen im Objektkoordinatensystem bestehen.

- Der Benutzer legt auf einem Referenzbild einen oder mehrere Orte im objektbezogenen Koordinatensystem fest (Zielpositionen), auf dem ein Therapie-Lichtimpuls appliziert werden soll. Die Steuereinheit unterstützt daraufhin die Applikation eines Therapie-Lichtimpulses auf eine vorgegebene Position im objektbezogenen Koordinatensystem des Objektes.

  ○ Während der Benutzer die Position des Zielstrahls manuell verändert zeigt die Steuereinheit die Zielposition im objektbezogenen Koordinatensystem als überlagerte Information im Live-Bild an.

  ○ Die Steuereinheit gibt genau dann die Auslösung eines Therapie-Lichtimpulses frei, wenn der Benutzer den Zielstrahl mit einer a priori festgelegten Genauigkeit auf die Zielposition gesteuert hat.

  ○ Die Steuereinheit löst genau dann selbsttätig einen Lichtimpuls aus, wenn sie den Zielstrahl selbsttätig auf die Zielposition im öbjektbezogenen Koordinatensystem gesteuert hat.

- Die Steuereinheit erzeugt ein Muster aus zwei oder mehreren Zielstrahlflecken auf dem Augenhintergrund, indem es den Zielstrahl in einer geeigneten zur Bewegung der Beobachtungsblende synchronisierten Weise bewegt und aktiviert. Die Position des Musters auf dem Augenhintergrund wird durch den Benutzer über eine Schnittstelle bestimmt oder durch die Steuereinheit ohne Einwirkung des Benutzers automatisch gesetzt. Die Auslösung des Therapie-Lichtimpulses erfolgt daraufhin auf den Positionen des durch den Zielstrahl erzeugten Musters. Eine Applikation von Therapie-Lichtimpulsen in solchen Mustern erhöht die Regelmäßigkeit der Impuls-Plazierung auf dem Augenhintergrund und damit die Reproduzierbarkeit des Therapieergebnisses. In einer weiteren Ausformung der Erfindung wird ein Muster nicht real auf dem Augenhintergrund erzeugt, sondern nur virtuell dem angezeigten Live-Bild überlagert. Die Bewegung des virtuellen Musters ist dabei an die Bewegung des Zielstrahlflecks auf dem Augenhintergrund geknüpft.

- Die Steuereinheit führt eine Stabilisierung des Live-Bildes bzgl. des Koordinatensystems des Objektes und der Anzeige aus, d. h. das Objekt-Koordinatensystem wird mit Bezug auf das Koordinatensystem der Anzeige fest gehalten.

- Die Steuereinheit führt eine Stabilisierung des Zielstrahlflecks im Objekt-Koordinatensystem aus, d. h. die Steuereinheit bewegt den Zielstrahl in einer geeigneten Weise, so dass er die gleichen Relativbewegungen ausführt wie das Objekt.

- Die Steuereinheit führt eine gleichzeitige Stabilisierung des Live-Bildes zum Koordinatensystem der Anzeige und die Stabilisierung des Zielstrahlflecks im Objekt-Koordinatensystem durch.

- Während der Durchführung einer therapeutischen Maßnahme mittels des Therapielichtstrahls führt die Steuereinheit eine Speicherung der Einwirkungsorte (der Koordinaten im objektbezogenen Koordinatensystem, an denen Lichtimpulse appliziert wurden) und der Parameter der Therapie-Lichtimpulse (wie Pulsdauer, Lichtleistung etc.) durch, so dass diese Informationen auch ohne sichtbare Spuren der Lichteinwirkung auf dem Objekt jederzeit durch Überlagerung der gespeicherten Informationen wieder auf Live- oder Standbildern sichtbar gemacht werden können.

- Nach der Applikation eines Therapie-Lichtimpulses wertet die Steuereinheit die Reflektivität des Gewebes an der Stelle der Therapie-Lichteinwirkung mittels Bildverarbeitung oder eine andere Messgröße aus, die mit der Temperatur des Gewebes während der Therapie-Lichtimpulsehwirkung und damit dem therapeutischen Effekt korreliert ist, und verändert die Parameter der Therapie-Lichtquelle für den nächsten Therapie-Lichtimpuls in geeigneter Weise, In einer weiteren Ausführung wird die Auswertung mittels Bildverarbeitung oder die Auswertung einer geeigneten Messgröße während der Applikation eines Therapie-Lichtimpulses ausgeführt und die Therapie-Lichtimpulseinwirkung beendet, sobald ein angestrebter Zustand des Gewebes erreicht ist.

Das erfindungsgemäße Ophthalmoskop ist zusätzlich oder alternativ zu den oben beschriebenen Therapiefunktionen zur Durchführung von Fluoreszenzmessungen eingerichtet, wie nachfolgend erläutert werden wird:

Wie eingangs beschrieben worden ist, werden Ophthalmoskope neben der Erzeugung von einfachen Farbbildern der Netzhaut auch für komplexere Bildgebungsverfahren verwendet wie z. B. für den qualitativen Nachweis der Fluoreszenz eines Farbstoffes, der in die Blutbahn des Patienten eingebracht wurde, in den Gefäßen der Netzhaut. Die besondere Herausforderung auf diesem Gebiet besteht im Nachweis der schwachen Fluoreszenz mit einer Optik, deren Lichtleitwert durch die besonderen Gegebenheiten bei der Beobachtung des Augenhintergrundes begrenzt ist. Eine besondere Herausforderung besteht darin, die Fluoreszenz von natürlicherweise im menschlichen Auge vorhandenen Farbstoffen (z. B. Lipofuscin) und ihre räumliche Verteilung nachzuweisen, deren Konzentration und damit deren Leuchtkraft nach Fluoreszenzanregung i. d. R. sehr gering ist.

In der Regel werden für eine Fluoreszenz-Messung mit einer breitbandigen Lichtquelle zwei Filter verwendet: ein Anregungsfilter für die Beleuchtung und ein Sperrfilter für die Beobachtung. Bei Verwendung einer schmalbandigen Lichtquelle (z. B. Laser, LED) kann auf den Anregungsfilter verzichtet werden. Die Transmissionskurven der Filter sind derartig ausgeführt, dass ihre maximale Transmission mit den Maxima der Absorptions- bzw. Emissionskurven der Farbstoffe übereinstimmt. Gleichzeitig muß der Sperrfilter jedoch effizient das gesamte Licht der Anregung unterdrücken, um ein hohes Signal-zu-Rausch-Verhältnis zu erhalten.

Das Fluoreszenzsignal eines Objektes ist in der Regel um Größenordnungen kleiner als z. B. das vom Objekt nach dem Lambertschen Gesetz gestreute Anregungslicht. Ein schwaches Meßsignal erfordert teure, empfindliche Sensoren, die dadurch bedingt eine niedrige Auflösung haben. Auf der anderen Seite kann die Lichteinstrahlung auf das Objekt gerade im Bereich der Augenheilkunde nicht beliebig erhöht werden, es bestehen vielmehr gerade bei kurzen sichtbaren Wellenlängen empfindlich niedrige Grenzwerte. Daher kann nicht auf diesem Weg das Meßergebnis verbessert werden.

Die in der Bildverarbeitung übliche Methode, das Signal-zu-Rausch-Verhältnis durch Mittelung mehrerer Bilder (Messungen) zu erhöhen, kann in der Augenheilkunde im Allgemeinen nicht angewendet werden, da sich das Objekt, das menschliche Auge, während der Beobachtung bewegt. Die Registrierung mehrerer Bilder aufeinander, d. h. die Bestimmung der Transformationen, die zwischen einzelnen Bildern einer Bildserie stattgefunden haben, könnte eine Mittelwertbildung ermöglichen. Bei der speziellen Anwendung der Fluoreszenzmessung im menschlichen Auge ist die Anwendung einer solchen Technologie jedoch nicht möglich, da die Fluoreszenzbilder aufgrund des schwachen Signals schlecht ausgesteuert und stark verrauscht sind.

Die hier vorgestellte Erfindung ist besonders für die Erstellung von Fluoreszenzmessungen des Augenhintergrundes geeignet, da sie in geeigneter Weise die Mittelung von möglicherweise stark verrauschten Bildern sich bewegender Objekte ermöglicht. In einer Vorrichtung zur Fluoreszenzmessung nach dem Stand der Technik wird am Sperrfilter der größte Teil des Lichtes, das vom zu beobachtenden Objekt ausgeht, absorbiert bzw. reflektiert.

Gemäß der Erfindung wird das Licht der Anregungslichtquelle für eine Verbesserung des Meßsignals verwendet. Dazu wird in den Beobachtungsstrahlengang ein dichroitischer Strahlteiler eingebracht, der das vom Objekt kommende Licht in den kurzwelligen Anteil des Anregungslichtes und den langwelligen Anteil der Fluoreszenzemission aufteilt. Das reflektierte Licht wird dabei auf einen zusätzlichen Sensor abgebildet.

Die Sensoren werden zeitlich synchronisiert betrieben, d. h. sie arbeiten mit gleicher Bildwiederholfrequenz und starten die Integrationszeit eines Bildes zum gleichen Zeitpunkt. Dadurch können zeitlich synchrone Bildpaare aufgenommen werden. Das Fluoreszenzbild ist dabei in der Regel schlecht ausgesteuert, während das Bild des gestreuten Anregungslichtes wesentlich besser ausgesteuert ist. Es ist daher vergleichsweise leicht, aus dem Bild des Anregungslichtes die geometrischen Transformationen, die durch die Bewegungen des Objektes zwischen den Einzelbildern einer Bildfolge entstanden sind, zu berechnen. Die inversen Transformationen werden dann auf die Fluoreszenzbilder angewendet, so dass diese durch Addition gemittelt werden können. Durch dieses Verfahren kann das Signal-zu-Rausch-Verhältnis mit jedem Bildpaar idealerweise um einen Faktor $\sqrt{2}$ verbessert werden.

In einer weiteren vorteilhaften Ausführungsform der Erfindung wird das Licht einer zusätzlichen Lichtquelle verwendet, die in einem Wellenlängenbereich emittiert, der außerhalb des Absorptions- und Emissionsbandes des fluoreszierenden Objektes liegt. Zweckmäßigerweise wird diese zusätzliche Lichtquelle in den Bleuchtungsstrahlengang eingekoppelt. In den Beobachtungsstrahlengang wird nun ein dichroitischer Strahlteiler eingebracht, der das vom Objekt kommende Licht in den kurzwelligen Anteil des Anregungslichtes und das Licht der zusätzlichen Lichtquelle einerseits und den langwelligen Anteil der Fluoreszenzemission andererseits aufteilt.

Die Verwendung einer zusätzlichen Lichtquelle bietet den Vorteil, dass unabhängig von den Anforderungen der Fluoreszenzmessung die Wellenlänge außerhalb der Absorptions- und Emissionsbanden frei gewählt werden kann und günstigerweise in einem Wellenlängenbereich gewählt wird, der eine stärkere Bestrahlung des Auges zuläßt. Durch die höhere Lichtintensität sind die Bilder, die zur Bestimmung der geometrischen Transformationen verwendet werden, besser ausgesteuert sind, dadurch kann die Mittelung der Fluoreszenzmessungen genauer ausgeführt werden.

Bevorzugte Ausführungsformen der Erfindung werden nachfolgend anhand der rein beispielhaften und keineswegs beschränkenden Zeichnungen erläutert werden. Es zeigt:

 Figur 1 eine schematische Darstellung eines Ophthalmoskops des Stands der Technik;

Figur 2 eine der Figur 1 ähnliche Darstellung einer ersten Ausführungsform des erfindungsgemäßen Ophthalmoskops;

Figuren 3a bis 3c drei vereinfachte Darstellungen eines Felds von fotosensitiven Pixeln eines elektronischen Sensors zu drei verschiedenen Zeitpunkten während eines Auslesevorgangs;

Figur 4a ein vereinfachtes Zeitdiagramm zur Erläuterung der Bedeutung der Belichtungszeit $t_{INT}$ einer Pixelzeile;

Figur 4b ein vereinfachtes Zeitdiagramm eines Triggersignals zum Starten eines Auslesevorgangs des elektronischen Sensors;

Figur 5 eine schematische Darstellung einer weiteren Ausführungsform des erfindungsgemäßen Ophthalmoskops mit einer Linienfokussieroptik und einem schwenkbar gelagerten Spiegel im Beleuchtungsstrahl;

Figur 6 eine schematische Darstellung einer weiteren Ausführungsform des erfindungsgemäßen Ophthalmoskops mit zwei Beobachtungsstrahlen für stereoskopische Abbildungen;

Figur 7 eine schematische Darstellung einer weiteren Ausführungsform des erfindungsgemäßen Ophthalmoskops zur gleichzeitigen Untersuchung des Augenvordergrunds;

Figur 8 eine um Therapiefunktionen erweiterte Ausführungsform des erfindungsgemäßen Ophthalmoskops;

Figur 9a und b zwei Querschnittsdarstellungen der Pupillenebene des menschlichen Auges beim Einsatz des Ophthalmoskops aus Figur 8;

Figur 10 ein Zeitdiagramm, das die Funktionsweise der zeitlichen Aktivierung des Zielstrahls im Ophthalmoskop der Figur 8 erläutert;

Figur 11 eine schematische Ansicht, die den im Zeitdiagramm aus Figur 10 erläuterten Ablauf im Bildfeld darstellt;

Figur 12 eine Ausführungsform des erfindungsgemäßen Ophthalmoskops für die gleichzeitige Messung von Fluoreszenzemissionen; und

Figur 13 eine erweiterte Ausbildung der Ausführungsform aus Figur 12, bei der eine zusätzliche Lichtquelle zur Verbesserung des Fluoreszenz-Meßergebnisses eingesetzt wird.

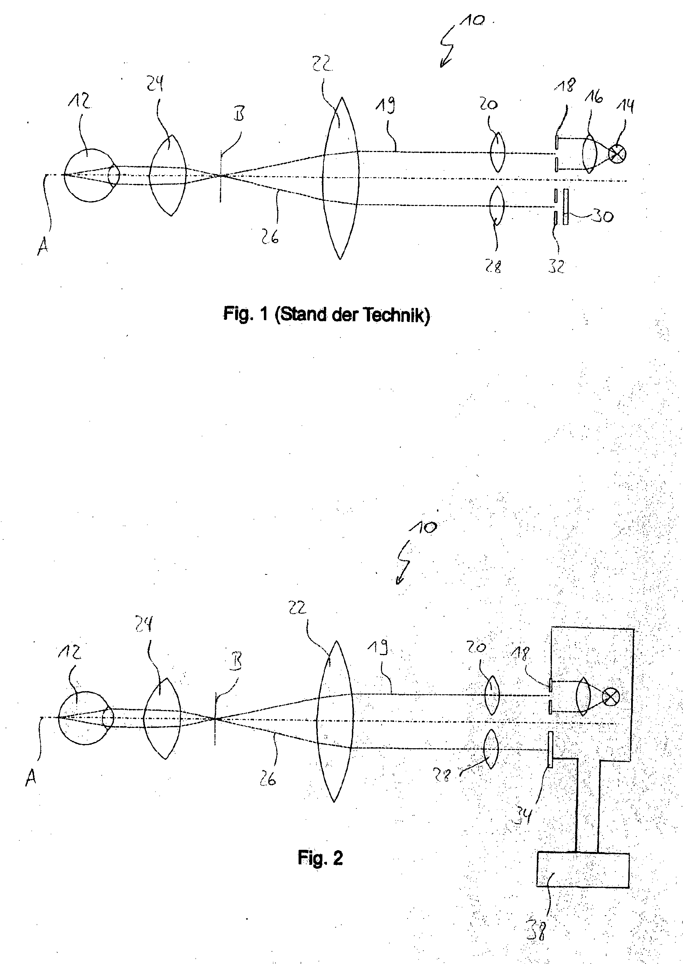

Figur 1 zeigt eine schematische Darstellung eines Ophthalmoskops 10 des Stands der Technik zur Untersuchung eines Auges 12 eines Patienten.

Das von einer Beleuchtungsvorrichtung 14 ausgesandte Licht wird mittels eines Kondensors 16 kollimiert und fällt auf eine Schlitzblende 18, die hin- und her oszilliert, und zwar in der schematischen Darstellung der Figur 1 in der Zeichenebene in einer Richtung senkrecht zur optischen Achse A des Ophthalmoskops 10. Die Schlitzblende 18 ist senkrecht zur Zeichenebene orientiert.

Auf diese Weise wird aus dem flächigen Lichtfleck, zu dem der Kondensor 16 das Licht der Beleuchtungsvorrichtung 14 kollimiert hat, eine Linie ausgekoppelt, deren Länge und Stärke von der Form der Schlitzblende 18 definiert wird, und deren Position von der aktuellen Lage der oszillierenden Schlitzblende abhängt.

Über weitere Linsen 20, 22 wird der Beleuchtungsstrahl 19 in eine Zwischenbildebene B fokussiert, aus der er über über eine ophthalmoskopische Linse 24 auf das Auge 12 abgebildet wird. Die schematische Darstellung der Figur 1 zeigt hierbei eine Abbildung auf den Augenhintergrund, beispielsweise für eine Netzhautuntersuchung.

Der aus der Reflexion des Beleuchtungsstrahls 19 am Augenhintergrund hervorgehende Beobachtungsstrahl bildet den Augenhintergrund über die ophthalmoskopische Linse 24 in die Zwischenbildebene B ab. Dieses Zwischenbild wird mittels der weiteren Linsen 22, 28 auf einen CCD-Sensor 30 fokussiert. Unmittelbar vor Erreichen des CCD-Sensors 30 muß der Beobachtungsstrahl 26 hierbei durch eine weitere Schlitzblende 32 gelangen, die vor dem CCD-Sensor synchron mit der Schlitzblende 18 im Strahlengang des Beleuchtungsstrahls 19 oszilliert.

Auf diese Weise wird mit Hilfe der Schlitzblende 32 sichergestellt, dass im wesentlichen nur der gewünschte Beobachtungsstrahl 26 den CCD-Sensor 30 erreicht, während Streulicht ausgeblendet wird. Derartiges Streulicht kann beispielsweise auf Reflexionen an der Augenvorderseite oder auch auf Streuungen an Glaskörpertrübungen des Auges 12 zurückzuführen sein.

Man erkennt in Figur 1, dass die oszillierende Schlitzblende 32 verhältnismäßig nah am CCD-Sensor 30 angeordnet ist. Dies ist unerläßlich, da mit zunehmendem Abstand zwischen Schlitzblende 32 und CCD-Sensor 30 die Unterdrückung störenden Streulichts verschlechtert wird. Allerdings führt diese Nähe dazu, dass sich Vibrationen von der oszillierenden Schlitzblende 32 auf den CCD-Sensor 30 übertragen können, beispielsweise über ein in den Figuren nicht dargestelltes Gehäuse des Ophthalmoskops 10. Dies führt zu grundsätzlichen Problemen bei der Handhabung des Ophthalmoskops 10, insbesondere verschlechtert sich die Schärfe des vom CCD-Sensor 30 gelieferten Bildes des Auges 12.

Figur 2 zeigt eine der Figur 1 ähnliche schematische Darstellung eines erfindungsgemäßen Ophthalmoskops 10, bei dem diese Probleme vermieden werden. Entsprechende Bauteile tragen in Figur 2 die gleichen Bezugzeichen wie in Figur 1. Das erfindungsgemäße Ophthalmoskop 10 gemäß der in Figur 2 dargestellten Ausführungsform unterscheidet sich von jenem des Stands der Technik dadurch, dass anstelle des CCD-Sensors 30 und der vor ihm hin- und her schwingenden Schlitzblende 32 ein elektronischer Sensor 34 im Beobachtungsstrahl 26 vorgesehen ist, der ein Feld von jeweils zeilenweise aktivierbaren und/oder auslesbaren fotosensitiven Pixeln umfaßt, die mittels einer elektronischen Steuerschaltung einzeln angesteuert werden können. Die elektronische Steuerschaltung ist im elektronischen Sensor 34 angeordnet und in den Figuren nicht dargestellt.

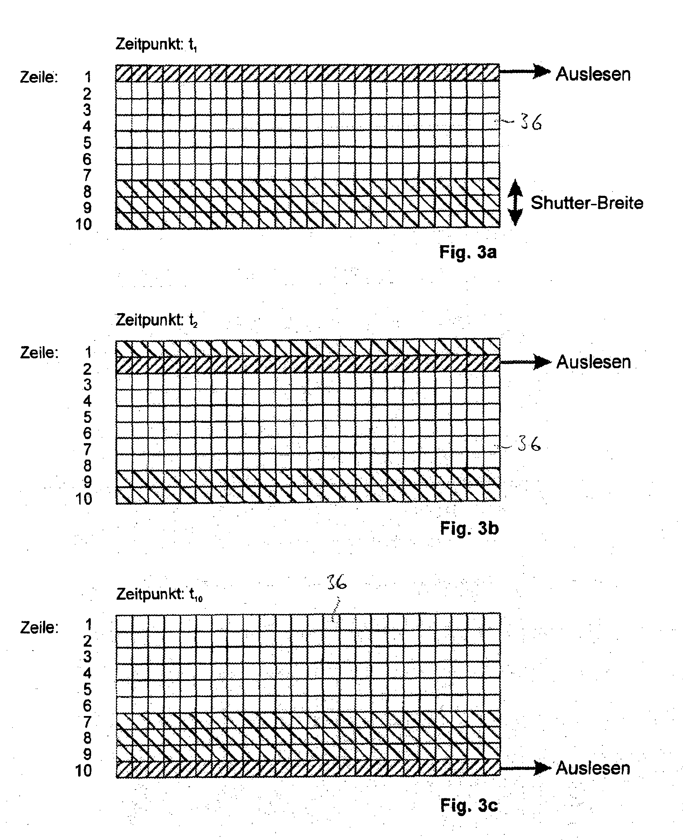

Die Funktionsweise des durch die elektronische Steuerschaltung kontrollierten elektronischen Sensors 34 wird nachfolgend anhand der Figuren 3a bis 3c erläutert werden:

Die Figuren 3a bis 3c zeigen eine schematische Vorderansicht eines Felds von fotosensitiven Pixeln eines vereinfacht dargestellten elektronischen Sensors 34. In der gezeigten vereinfachten Ausführungsform umfaßt dieses Feld von fotosensitiven Pixeln insgesamt 10 Pixelzeilen, die mit 1,2, ..., 10 durchnumeriert sind. Jede Pixelzeile umfaßt in den Figuren 3a bis 3c vierundzwanzig Pixel, die mittels fotosensitiver Elemente abhängig von der einfallenden Lichtmenge Ladungen erzeugen. Jedem Pixel 36 ist eine eigene Verstärkerelektronik zugeordnet, die die im Pixel 36 generierte elektrische Ladung in ein jeweiliges Spannungssignal umwandelt. Die Funktionsweise derartiger elektronischer Sensoren mit sogenannten "aktiven Pixeln", insbesondere CMOS-Sensoren, ist an sich bekannt und wird hier nicht weiter erläutert werden.

Mit Hilfe der im elektronischen Sensor 34 vorgesehenen elektronischen Steuerschaltung kann jede Pixelzeile in einen von drei Betriebszuständen geschaltet werden:

a) Einen deaktivierten Modus, in dem die Pixelzeile trotz Lichteinfalls keine Ladungen integriert und somit kein Spannungssignal erzeugt; in Figur 3a befinden sich die Pixelzeilen 2, 3, ..., 7 in diesem deaktivierten Modus, was durch leere Pixel 36 in diesen Pixelzeilen angedeutet wird.

b) Einen aktiven Modus, in dem Lichteinfall zur Erzeugung eines kontinuierlich ansteigenden Spannungssignals führt; in Figur 3a befinden sich die Pixelzeilen 8, 9 und 10 in diesem Betriebszustand, was durch eine einfache Schraffur dieser Pixelzeilen dargestellt ist.

c) Einen Auslesemodus, in dem eine Pixelzeile ausgelesen wird, d.h. das in einem bestimmten vorangegangenen Belichtungszeitraum aufgebaute Spannungssignal von der elektronischen Steuerschaltung abgerufen wird; die Pixelzeile wird anschließend gelöscht, d.h. das Spannungssignal wieder auf den Wert Null zurückgesetzt. In Figur 3a befindet sich nur die Pixelzeile 1 in diesem Auslesemodus, angedeutet durch die dichte Schraffur.

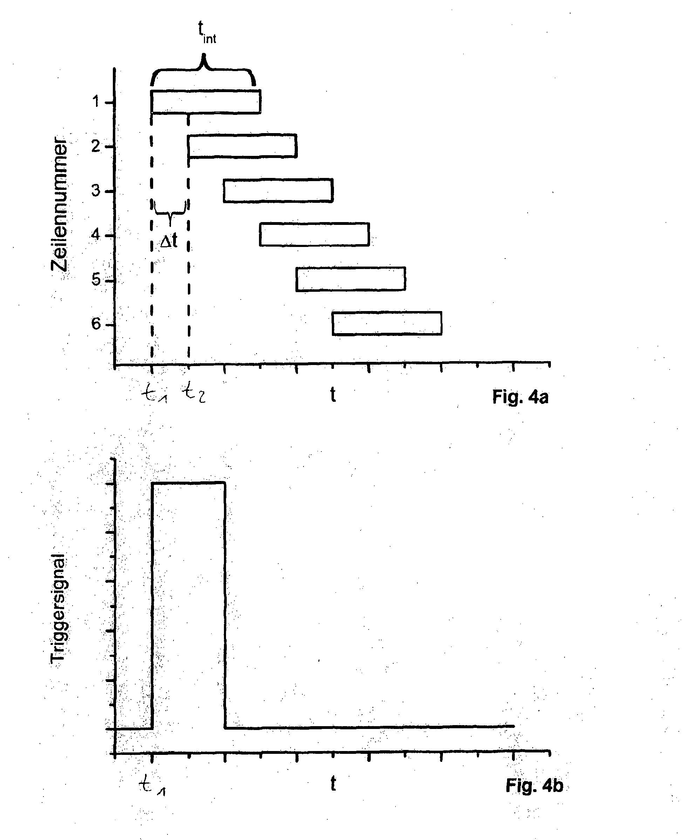

Der durch die elektronische Steuerschaltung kontrollierte Auslesevorgang des elektronischen Sensors 34 wird nachfolgend anhand der Figuren 3a bis 3c, 4a und 4b erläutert werden:

Zu einem in den Zeitdiagrammen der Figuren 4a und 4b gekennzeichneten Startzeitpunkt $t_1$ empfängt die elektronische Steuerschaltung von einer elektronischen Kontrolleinheit 38, beispielsweise einem zentralen Steuercomputer, ein Triggersignal, beispielsweise ein TTL-Signal. Die ansteigende Flanke des in Figur 4b dargestellten Triggersignals startet einen vollständigen Auslesedurchlauf des elektronischen Sensors 34, indem die Pixelzeile Nummer 1 vom inaktiven Zustand zunächst in den Auslesezustand und anschließend in den aktiven Zustand geschaltet wird, d.h. Lichteinfall führt zum Aufbau eines Spannungssignals in jedem Pixel der ersten Pixelzeile. Die erste Pixelzeile bleibt während einer Belichtungszeitdauer $t_{INT}$ in diesem aktiven Zustand und wird zu einem entsprechenden Zeitpunkt $t_1$ des nächsten Auslesedurchlaufs von der elektronischen Steuerschaltung ausgelesen.

Nach Ablauf einer einstellbaren Zeitverzögerung $\Delta t$ nach dem Startzeitpunkt $t_1$ wird auch die zweite Pixelzeile ausgelesen und in den aktiven Zustand geschaltet, und um eine weitere Zeitverzögerung $\Delta t$ später auch die dritte Pixelzeile. Bei dem in Figur 4a gezeigten Fall wird $t_{INT}=3x\Delta t$ gewählt, so dass sich zum Zeitpunkt des Auslesens und Aktivierens der vierten Pixelzeile $t_4=t_1+t_{INT}=t_1+3x\Delta t$ momentan auch die zweite und die dritte Pixelzeile im aktiven Modus befinden, wie in Figur 3a gezeigt ist, wohingegen die erste Pixelzeile gerade deaktiviert wird.

Nach dem Auslesen der ersten Pixelzeile zum Zeitpunkt $t_1$ wird diese aktiviert, und eine weitere Zeitverzögerung $\Delta t$ später, also zu einem Zeitpunkt $t_2=t_1+\Delta t$, wird die zweite Pixelzeile ausgelesen, die ab dem Zeitpunkt $t_2$ des vorhergehenden Durchlaufs während der Zeitdauer $t_{INT}$ belichtet wurde. Auf diese Weise schreitet der Auslesevorgang jeweils in zeitlichen Abständen entsprechend der Zeitverzögerung $\Delta t$ um eine Pixelzeile des elektronischen Sensors 34 weiter, bei dem in Figuren 3a bis 3c gezeigten Beispiel von Pixelzeile Nummer 1 bis zur Pixelzeile Nummer 10. Die Wahl von $t_{INT}=3x\Delta t$ führt dazu, dass zum Zeitpunkt des Auslesens der n-ten Pixelzeile drei benachbarte Pixelzeilen aktiv sind, nämlich die Pixelzeilen n-1, n-2 und n-3. Dies definiert die effektive Breite der elektronischen Schlitzblende, die sich in den Figuren 3a bis 3c gleichsam von oben nach unten über das Feld von fotosensitiven Pixeln 36 bewegt. Man beachte, dass die in einer Pixelzeile im Zeitraum $t_{INT}$ aufgebaute Spannung erst im nächsten Durchlauf der elektronischen Auslesung abgefragt wird, wenn diese Pixelzeile in den Auslesemodus geschaltet wird.

Die für einen vollständigen Durchlauf benötigte Gesamtzeit T entspricht dem Produkt aus der Zahl N der Pixelzeilen (im Beispiel der Figuren 3a bis 3c ist N=10) und der Zeitverzögerung $\Delta t$, also $T = N \times \Delta t$. Durch die Wahl der drei Parameter $\Delta t$, $t_{INT}$ und N können somit die Bildwiederholungsrate 1/T sowie die effektive Breite der elektronischen Spaltblende flexibler eingestellt werden als die entsprechenden Parameter einer mechanisch oszillierenden Schlitzblende 32 im Stand der Technik gemäß Figur 1.

Die elektronische Kontrolleinheit 38 ist dazu ausgelegt, das in Figur 4b dargestellte Triggersignal zur Synchronisierung der jeweils aktiven Pixelzeilen mit der Bewegung der Schlitzblende 18 im Beleuchtungsstrahl einzusetzen. Zum Zeitpunkt $t_1$ beispielsweise (siehe Figur 3a) sind die Pixelzeilen 8, 9 und 10 aktiv. Der Zeitpunkt $t_1$ muß daher derart gewählt sein, dass er einem Zeitpunkt entspricht, an dem die Schlitzblende 18 sich am unteren Umkehrpunkt ihrer Schwingungsbewegung befindet. Die in den Figuren 3a bis 3c und 4a dargestellte Abfolge von Pixelzeilen-Aktivierungsvorgängen von oben (in Figur 3a wird Pixelzeile Nummer 1 ausgelesen und unmittelbar anschließend aktiviert) nach unten (in Figur 3c wird Pixelzeile 10 ausgelesen und unmittelbar anschließend aktiviert) verläuft dann synchron zu einer Bewegung der Schlitzblende 18 von oben nach unten in Figur 2.

Da, wie in den Figuren 3a bis 3c und 4a gezeigt, bei diesem Prinzip des "rolling shutter" gleichsam eine elektronische Blende stets von oben nach unten über das Pixelfeld läuft, dürfen nur solche Bilder des elektronischen Sensors 34 verarbeitet werden, die einer hierzu synchronen Abwärtsbewegung des Beleuchtungsstrahls 19 entsprechen. Diejenigen Schwingungsphasen, in denen sich die Schlitzblende 18 in Figur 2 aufwärts bewegt, sind nicht verwertbar. Aus diesem Grund wird nach einem vollständigen Auslese-Durchlauf des elektronischen Sensors 34, in dem die im vorangegangenen Durchlauf in den einzelnen Pixelzeilen aufgebauten Spannungssignale ausgelesen werden, die Abgabe des nächsten Triggersignals durch die elektronische Kontrolleinheit 38 verzögert, bis die Schlitzblende 18 wieder ihren oberen Umkehrpunkt erreicht hat.

Eine weitere Verbesserung des durch den elektronischen Sensor 34 gelieferten Bilds des Auges 12 läßt sich dadurch erreichen, dass nicht nur der Auslese-Startzeitpunkt $t_1$ mit dem oberen Umkehrpunkt der schwingenden Schlitzblende 18 synchronisiert wird, sondern auch die Abwärtsbewegung der elektronischen Blende in den Figuren 3a bis 3c auf die entsprechende Abwärtsbewegung der Schlitzblende 18 im Beleuchtungsstrahl 19 abgestimmt wird. Wenn beispielsweise die Schlitzblende 18 sinusförmig im Beleuchtungsstrahl 19 hin- und her schwingt, ist ihre Geschwindigkeit, mit der sie durch den Beleuchtungsstrahl 19 fährt, am oberen und am unteren Umkehrpunkt der Schwingung jeweils gleich Null, während sie in der Mitte zwischen den beiden Umkehrpunkten ein Maximum erreicht. Dies kann im elektronischen Sensor 34 dadurch berücksichtigt werden, dass die Zeitverzögerung $\Delta t$ abweichend von Figur 4a nicht konstant gewählt wird, sondern als Funktion der jeweiligen Pixelzeile an den oberen und unteren Rändern des Pixelfelds (beispielsweise im Bereich der Pixelzeilen 1 und 10) größer gewählt wird als in der Mitte des Pixelfelds (in der Nähe der Pixelzeilen 5 und 6).

Dieser zusätzliche elektronische Aufwand, der sich ergibt, wenn man die Bewegung des "rolling shutter" des elektronischen Sensors 34 an die Bewegung der mechanischen Schlitzblende 18 anpassen möchte, läßt sich vermeiden, wenn man in umgekehrter Weise den in den Figuren 3a bis 3c, 4a und 4b dargestellten zeitlichen Ablauf der elektronischen Blende beibehält und die Bewegung der mechanischen Schlitzblende hieran anpaßt. Beispielsweise kann die elektronische Kontrolleinheit 38 einen in den Figuren nicht dargestellten Antriebsmechanismus der Schlitzblende 18 derart kontrollieren, dass die Schlitzblende 18 eine Art "Sägezahntrajektorie" durchläuft, also mit konstanter Geschwindigkeit von ihrem oberen Umkehrpunkt zu ihrem unteren Umkehrpunkt bewegt wird und anschließend deutlich schneller an ihren oberen Umkehrpunkt zurückgestellt wird.

Je nach gewünschter Bildwiederholungsfrequenz kann sich eine derartige sägezahnförmige Bewegung eines ausgedehnten Blechs, in dem Schlitzblende 18 vorgesehen ist, jedoch als schwierig erweisen.

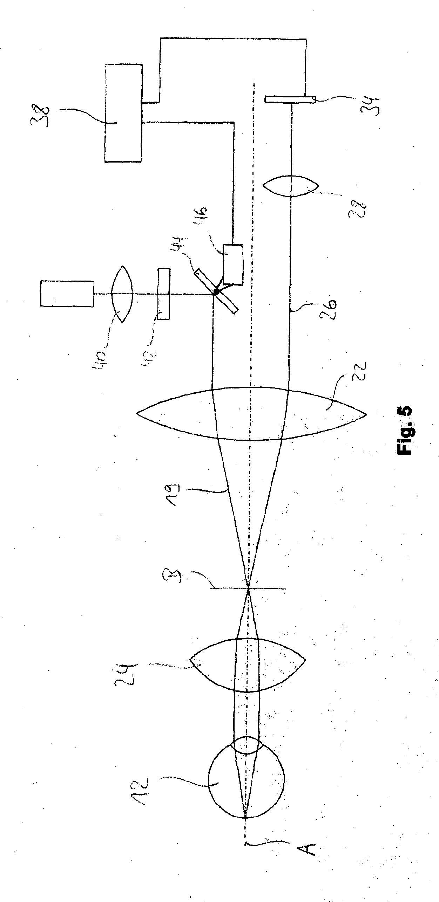

In einer in Figur 5 dargestellten bevorzugten Ausführungsform des erfindungsgemäßen Ophthalmoskops 10 ist daher in Form einer Linienfokussieroptik auch die Erzeugung des Beleuchtungsstrahls 19 gegenüber dem Stand der Technik gemäß Figur 1 abgeändert, um die Synchronisierung mit der elektronischen Blende im elektronischen Sensor 34 zu erleichtern:

In Figur 5 sind Komponenten, die jenen der in Figur 2 dargestellten Ausführungsform entsprechen, mit gleichen Bezugszeichen bezeichnet. Insbesondere wird auch bei der bevorzugten Ausführungsform gemäß Figur 5 ein elektronischer Sensor 34 mit den oben beschriebenen Eigenschaften zur Erfassung des Beobachtungsstrahls verwendet.

In dieser Ausführungsform jedoch wird als Beleuchtungsvorrichtung 14 eine punktförmige Lichtquelle, z.B. ein Laser oder das Ende eines Lichtwellenleiters eingesetzt. Das von ihm ausgehende Licht wird durch einen Kondensor 40 in ein paralleles Strahlenbündel überführt und auf eine Zylinderlinse 42 gerichtet. In Strahlrichtung hinter der Zylinderlinse 42 ist ein schwenkbar gelagerter Spiegel 44 angeordnet, der den Beleuchtungsstrahl 19 annähernd parallel zur optischen Achse A in Figur 5 nach links in Richtung auf die Linse 22 reflektiert, welche sich in der fokussierenden Ebene der Zylinderlinse 42 befindet. Die Zylinderlinse 42 ist hierbei derart positioniert, dass der von ihr erzeugte Linienfokus senkrecht zur Zeichenebene der Figur 5 orientiert ist.

Der Spiegel 44 ist schwenkbar an einer Scanvorrichtung 46 gelagert, die von der elektronischen Kontrolleinheit 38 gesteuert wird, welche gleichzeitig die elektronische Steuerschaltung des elektronischen Sensors 34 kontrolliert. Die elektronische Kontrolleinheit 38 ist dazu ausgelegt, die Scanvorrichtung 46 derart zu kontrollieren, dass sie mittels einer Drehung des Spiegels 44 in der Zeichenebene der Figur 5 den Beleuchtungsstrahl 19 synchron zur Bewegung der elektronischen Blende im elektronischen Sensor 34 über das Auge 12 scannt. Bei dem in den Figuren 3a bis 3c gezeigten kontinuierlichen linearen Abwärtsverlauf der elektronischen Blende kann der Spiegel 44 eine Sägezahntrajektorie durchlaufen, indem er beispielsweise ausgehend von der in Figur 5 gezeigten Stellung schrittweise entgegen dem Uhrzeigersinn gedreht wird, und schließlich, sobald die elektronische Blende im elektronischen Sensor 34 die untere Pixelzeile erreicht hat, beispielsweise die Pixelzeile Nummer 10 in Figur 3c, schlagartig im Uhrzeigersinn in seine Ausgangsposition gemäß Figur 5 zurückgestellt wird.

Ein derartiger schwenkbar gelagerter Spiegel weist in der Praxis ein sehr geringes Trägheitsmoment auf, so dass er nicht nur bei hohen Frequenzen, sondern generell in einem großen Frequenzbereich eingesetzt werden kann. Die Kombination eines derartigen Spiegels mit einem "rolling shutter" gemäß Figur 5, die somit eine freie Wahl der Bildwiederholrate und eine externe Synchronisation mit Hilfe der elektronischen Kontrolleinheit 38 ermöglicht, erlaubt daher eine optimale Anpassung der Beleuchtung des Auges 12 an die jeweils gewünschte Beobachtung. Durch die geringe Trägheit des Spiegels 44 werden Vibrationen im erfindungsgemäßen Ophthalmoskop 10 nahezu vollständig vermieden.

Der Einsatz einer kohärenten Lichtquelle, beispielsweise eines Lasers, erlaubt die Erzeugung eines sehr schmalen Beleuchtungsspalts. Wählt man gleichzeitig eine minimale elektronische Spaltbreite von einer einzigen Pixelzeile entprechend einer Höhe eines Pixel 36 von ca. 5μm, so kann man eine senkrecht zur Spaltausdehnung konfokale Abbildung erzielen und somit dreidimensionale Untersuchungen des Auges 12 vornehmen.

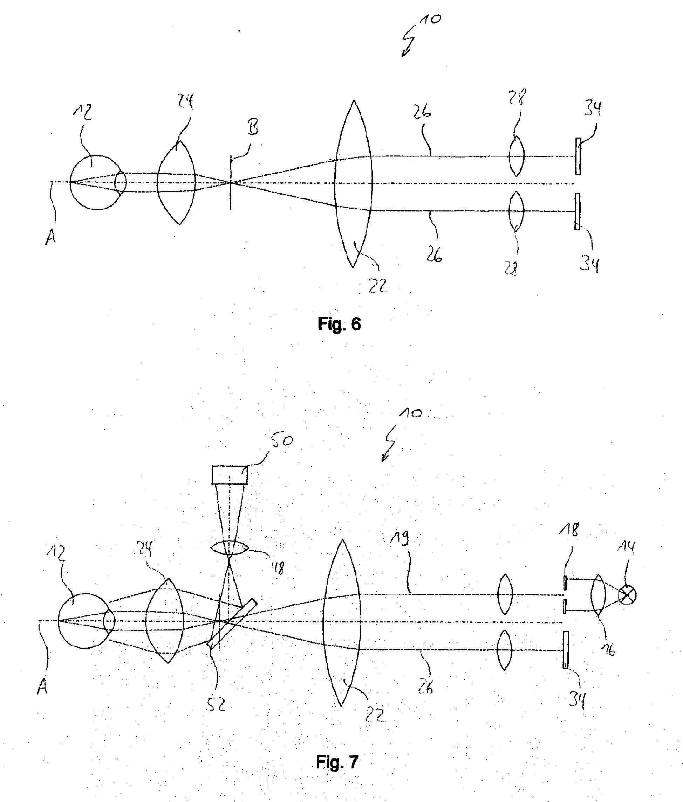

Figur 6 zeigt eine schematische Darstellung einer weiteren Ausführungsform des erfindungsgemäßen Ophthalmoskops 10 mit zwei Beobachtungsstrahlen 26 für stereoskopische Abbildungen. Der Beleuchtungsstrahlengang liegt hierbei außerhalb der Zeichenebene und ist in Figur 6 nicht dargestellt. In dieser Ausführungsform sind zwei benachbarte elektronische Sensoren 34 vorgesehen, um stereoskopische Aufnahmes des Auges

12 anzufertigen. Der Beleuchtungsstrahlengang kann hierbei entweder wie in Figur 2 gewählt sein, d.h. mit einer Schlitzblende 18, die vor einer Beleuchtungsvorrichtung 14 hin- und her oszilliert, oder wie in Figur 5 gezeigt, d.h. mit einer Linienfokussieroptik im Beleuchtungsstrahl 19. Die Vorteile der vorliegenden Erfindung, nämlich die Vermeidung mechanisch hin- und her oszillierender Schlitzblenden, kommen bei der Ausführungsform der Figur 6 besonders deutlich zum Tragen, denn bei entsprechenden Ophthalmoskopen 10 des Stands der Technik mit der Möglichkeit der stereoskopischen Beobachtung sind insgesamt drei hin- und her oszillierende Schlitzblenden erforderlich, nämlich eine Schlitzblende im Beleuchtungsstrahl 19 sowie zwei Schlitzblenden 32 in den beiden zueinander im wesentlichen parallelen Beobachtungsstrahlen. Dies führt gerade bei stereoskopischen Ophthalmoskopen 10 des Stands der Technik zu ausgeprägten Vibrationsproblemen, die durch die Erfindung vermieden werden.

Figur 7 zeigt eine weitere Ausführungsform des erfindungsgemäßen Ophthalmoskops 10 mit einer zusätzlichen Abbildungseinheit, die den Augenvordergrund durch die ophthalmoskopische Linse 24 und eine weitere Linse 48 auf einen separaten Bildsensor 50 abbildet. Beim separaten Bildsensor 50 handelt es sich vorzugsweise ebenfalls um einen elektronischen Sensor mit aktiven Pixeln, beispielsweise einen CMOS-Sensor, wie er vorstehend ausführlich beschrieben wurde.

Die Trennung des Strahlengangs von Augenhintergrund- und Augenvordergrundbeobachtung wird in dieser Ausführungsform durch einen Strahlteiler 52 vorgenommen, wobei vorzugsweise ein Folienstrahlteiler (pellicle beam splitter) zum Einsatz kommt.

Der Augenvordergrund wird in dieser Ausführungsform mit einer in Figur 7 nicht dargestellten separaten Lichtquelle beleuchtet, die direkt vor dem Auge 12 positioniert ist und idealerweise im Infraroten emittiert.

Ansonsten entspricht die Ausführungsform der Figur 7 jener der Figur 2, d.h. mit einer mechanisch oszillierenden Schlitzblende 18 im Beleuchtungsstrahl 19. Selbstverständlich kann jedoch auch bei der Ausführungsform der Figur 7 die anhand der Figur 5 erläuterte Linienfokussieroptik zum Einsatz gelangen.

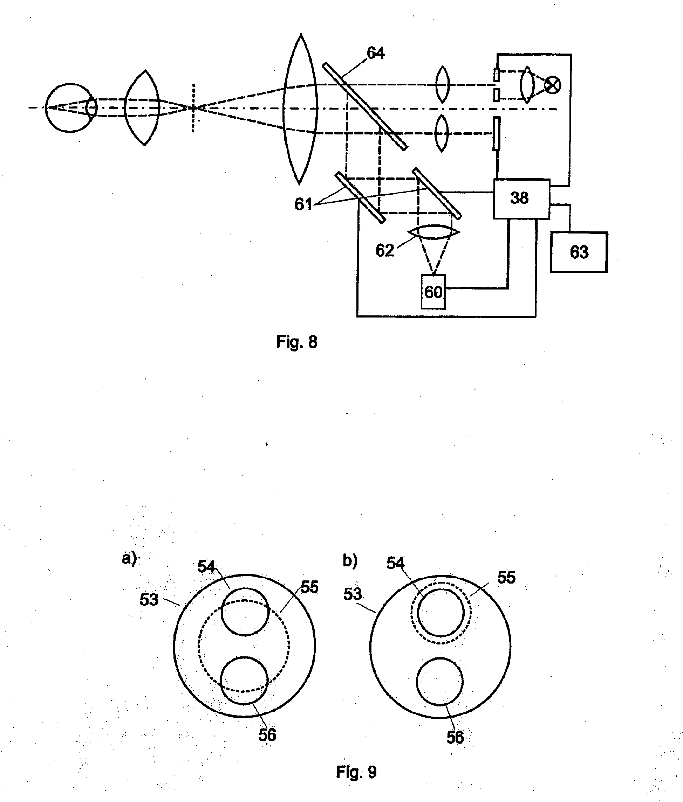

Figur 8 zeigt eine weitere Ausführungsform des erfindungsgemäßen Ophthalmoskops, die ausgehend von der Ausführungsform der Figur 2 um Therapiefunktionen erweitert worden ist. Hierzu ist eine weitere Lichtquelle 60 vorgesehen, die einen Therapie-Lichtstrahl erzeugt, welcher mittels einer Linse 62 kollimiert und über einen Strahlteiler 64 koaxial zur Hauptachse des Ophthalmoskops in den Strahlengang eingekoppelt wird. Der Therapie-Lichtstrahl wird dabei über zwei Spiegel 61 umgelenkt, die jeweils um eine zur Zeichenebene der Figur 8 senkrechte und eine in der Zeichenebene liegende Achse schwenkbar gelagert sind. Die elektronische Kontrolleinheit 38 enthält auch eine Steuereinheit, die die Schwenkbewegung der Spiegel 61 basierend auf Sollwerten kontrolliert, die ein Benutzer mittels einer Schnittstelle 63, beispielsweise eines Joysticks, eingeben kann.

Figur 9a zeigt eine schematische Querschnittsdarstellung der Pupillenebene des Auges eines Patienten im Fall der Verwendung des Ophthalmoskops aus Figur 8. Hierbei bezeichnet Bezugsziffer 53 die Pupille des Auges 12, Bezugsziffer 54 bezeichnet die Pupille des Beleuchtungsstrahlengangs, und Bezugsziffer 56 bezeichnet die Pupille des Beobachtungsstrahlengangs. Die Lage des zusätzlich mit Hilfe des Strahlteilers 64 eingekoppelten Therapie-Lichtstrahls ist in Figur 9a gestrichelt eingezeichnet und mit Bezugsziffer 55 bezeichnet. Entsprechend der Einkopplung des Therapie-Lichtstrahls in Figur 8 liegt der Therapie-Lichtstrahl 55 in Figur 9a zentral, d.h. auf der Hauptachse des erfindungsgemäßen Ophthalmoskops 10.

Alternativ kann der Therapie-Lichtstrahl mit Hilfe der Spiegel 61 und des Strahlteilers 64 auch dezentral, beispielsweise koaxial zum Beleuchtungsstrahlengang, eingekoppelt werden. Die entsprechende Querschnittsdarstellung der Pupillenebene des Patienten ist in Figur 9b gezeigt.

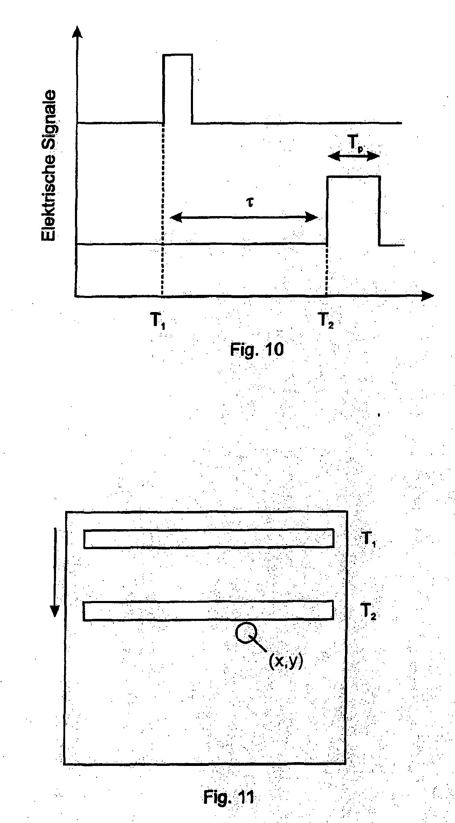

Figur 10 zeigt ein Zeitdiagramm, das die Funktionsweise der zeitlichen Aktivierung des Zielstrahls erläutert. Der Zeitpunkt $T_1$ stellt den Startpunkt der Blendenbewegung an einem Ende des Bildfeldes dar. Er entspricht zweckmäßigerweise dem oben erläuterten Zeitpunkt $t_1$, zu dem die erste Pixelzeile des elektronischen Sensors 34 ausgelesen wird. Somit ist auch der Zeitpunkt $T_1$ durch die in Figur 4b dargestellte ansteigende Flanke eines Rechteckimpulses definiert. Nach einer zeitlichen Verzögerung $\tau$, die sich wie oben beschrieben aus der Position des Zielstrahlflecks im Bildfeld (oder durch damit verbundene Größen) berechnen läßt, wird der Zielstrahl dann während eines Zeitraums $T_p$ zu einem Zeitpunkt $T_2$ aktiviert, was in Figur 10 wiederum durch einen Rechteckimpuls angedeutet ist.

Figur 11 stellt den im Zeitdiagramm der Figur 10 erläuterten Ablauf im Bildfeld dar. Zum Zeitpunkt $T_1$ befinden sich die Blenden am Startpunkt ihrer Bewegung. In Figur 11 ist der Startpunkt als am oberen Rand des Bildfeldes befindlich dargestellt. Die Bewegung der Blenden erfolgt vom Zeitpunkt $T_1$ an abwärts über das Bildfeld, wie durch einen Pfeil in Bewegungsrichtung angedeutet ist. Zum Zeitpunkt $T_2$ hat der untere Rand der Blende des erfindungsgemäßen Ophthalmoskops 10 diejenige Position (x, y) im Bildfeld erreicht, an der sich der Zielstrahlfleck befindet. Zu diesem Zeitpunkt wird daraufhin der Zielstrahl für die Zeitdauer $T_p$ aktiviert. Anders ausgedrückt wird das Einstrahlen des Zielstrahls auf diejenigen kurzen Zeiträume beschränkt, in denen die Blenden des' Ophthalmoskops 10 tatsächlich die Beobachtung des gewünschten Augenbereichs erlauben. Außerhalb dieser kurzen Zeiträume ist der Zielstrahl ausgeschaltet, wodurch störende Reflexe minimiert werden. Die Synchronisation dieses gepulsten Zielstrahls mit den Blenden erfolgt wiederum mit Hilfe der elektronischen Kontrolleinheit 38.

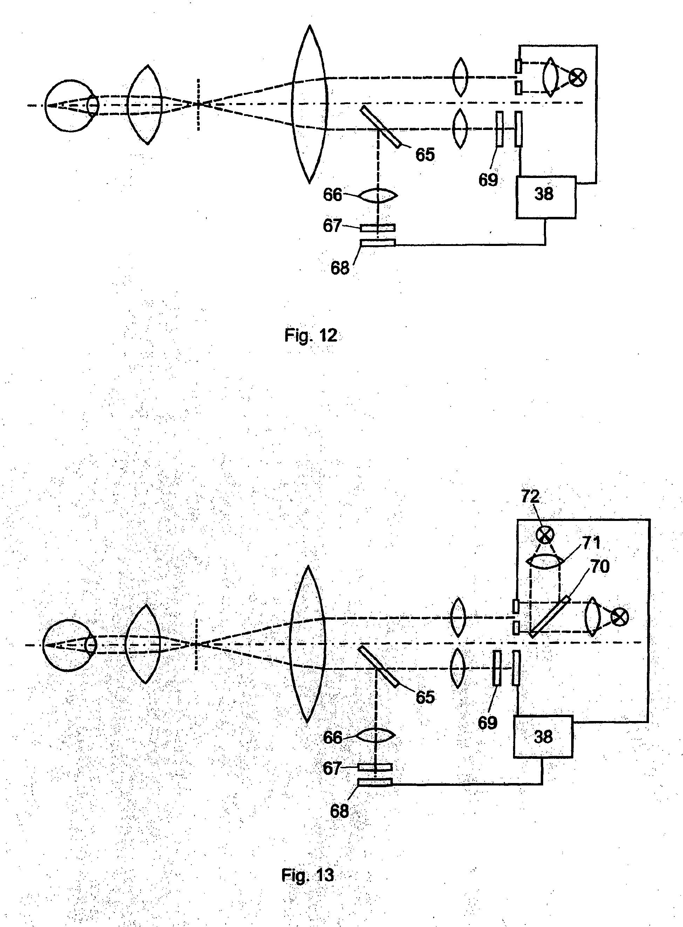

Figur 12 zeigt eine weitere Ausführungsform des erfindungsgemäßen Ophthalmoskops für die Messung von Fluoreszenzemissionen. Hierbei ist ein Strahlteiler 65 in den Beobachtungsstrahlengang eingebracht, der wellenlängenabhängig einen Teil des vom Objekt stammenden Lichts über eine Linse 66 auf einen zweiten Sensor 68 reflektiert. Der Strahlteiler 65 fungiert dabei gleichzeitig als Sperrfilter für die Fluoreszenzmessung. Die Filterwirkung des Strahlteilers kann durch zusätzliche Filter in Transmission 67 und 69 verbessert werden. Die elektronische Kontrolleinheit 38 enthält hierbei eine Steuereinheit, welche sicherstellt, dass die Sensoren 68 und 34 synchron ausgelesen werden und damit zwei Bilder zum gleichen Zeitpunkt aufgenommen werden.

Figur 13 stellt eine Weiterentwicklung der Ausführungsform der Figur 12 dar, bei der eine zusätzliche Lichtquelle 72 zur Verbesserung des Fluoreszenz-Meßergebnisses eingesetzt wird. Das Licht dieser zusätzlichen Lichtquelle 72 wird über einen Kondensor 71 kollimiert und über einen Strahlteiler 70 in den Beleuchtungsstrahlengang eingekoppelt. Die Lichtquelle 72 emittiert Licht in einem Wellenlängenbereich, der außerhalb der Absorptions- und Emissionsbanden des Fluoreszenzfarbstoffes liegt.

Die Erfindung ist nicht auf die als nicht beschränkende Beispiele vorgestellten Ausführungsformen beschränkt. So versteht sich beispielsweise, dass der anhand der Figuren 3a bis 3c erläuterte elektronische Sensor 34 nicht nur die dort aus Gründen der Einfachheit gezeigten zehn Pixelzeilen aufweist, sondern in der Praxis mehr als tausend Pixelzeilen.

Ferner versteht sich, dass der konkrete Verlauf der Strahlengänge des Beleuchtungsstrahls 19 sowie des Beobachtungsstrahls 26 bzw. der Beobachtungsstrahlen 26 im Falle stereoskopischer Abbildungen durch bekannte optische Elemente variiert werden kann. Ebenso versteht es sich, dass weitere Lichtstrahlen in die vorgestellten Strahlengänge eingekoppelt werden können, beispielsweise zu Therapie- oder Diagnosezwecken, wie es aus dem Stand der Technik grundsätzlich bekannt ist.

In all diesen abgewandelten Ausführungsformen bietet die Erfindung nicht nur die oben erläuterten Vorteile einer deutlichen Verringerung bzw. vollständigen Beseitigung mechanischer Vibrationen aufgrund hin- und her schwingender Schlitzblenden, sondern zahlreiche weitere Vorteile:

a) Wie bereits betont wurde, befindet sich die elektronische Blende beim elektronischen Sensor 34 in dessen Bildebene, so dass die Ausblendung von Streulicht optimiert wird.

b) Kontinuierliche Bildgebungsverfahren setzen eine ausreichend hohe Bildwiederholungsfrequenz von mehr als 10Hz voraus, um einen flüssigen Bewegungseindruck beim Beobachter zu erzeugen. Die im Stand der Technik eingesetzten Schlitzblenden müssen also mit hoher Frequenz schwingen, was in der Praxis nur mit einem mechanischen Oszillator in Resonanz zu erreichen ist. Dies setzt wiederum eine exakte und auf geringe Abweichungen äußerst empfindliche Abstimmung der Eigenfrequenz des Oszillators mit der Bildwiederholungsfrequenz des CCD-Sensors 30 voraus. Somit ist die Bildwiederholfrequenz für einen gegebenen Oszillator fest. Beim erfindungsgemäßen Ophthalmoskop 10 hingegen, insbesondere in der Ausführungsform gemäß Figur 5, die ohne jegliche mechanische hin- und her schwingende Schlitzblende arbeitet, ist keine derartige Beschränkung durch eine Resonanzbedingung gegeben.

c) Beim Ophthalmoskop des Stands der Technik ist die Spaltbreite für eine gegebene mechanisch schwingende Blende unveränderlich. Viele Anwendungen verlangen jedoch nach einer feinstufigen Variation der Spaltbreite und damit der effektiven Belichtungszeit. Beispielsweise ist eine Variation der Schlitzbreite oder der Bildwiederholfrequenz und damit der effektiven Belichtungszeit wünschenswert, um auf unterschiedliche Anwendungsfälle einzugehen: bei Angiographie des Augenhintergrunds muß die schwache Fluoreszenzemission eines Farbstoffes detektiert werden, was eine möglichst lange Belichtungszeit erfordert. Dies ist durch Einstellen der Parameter des elektronischen Sensors 34, insbesondere die Parameter N, $\Delta t$ und $t_{INT}$, problemlos möglich.

d) Beim Ophthalmoskop des Stands der Technik beträgt die Frequenz des Oszillators für den Antrieb der Schlitzblende in der Praxis ein Vielfaches der Bildwiederholfrequenz des CCD-Sensors, da mechanische Oszillatoren mit geringer Frequenz zum einen ungenau in ihrer Schwingfrequenz abzustimmen sind und zum anderen auch zu besonders unangenehmen Vibrationen des gesamten Gerätes führen. Dies führt zu Doppelbelichtungen auf dem CCD-Sensor 30 und damit zu Bildunschärfe und Bewegungsartefakten durch schnelle Bewegung des Auges 12 oder sonstigen untersuchten Objekts. Die Verwendung von Bildsensoren, die nach einem der gängigen Video-Standards (PAL, NTSC) zwei Halbbilder, die zu unterschiedlichen Zeitpunkten aufgenommen werden, zu einem Vollbild zusammenfügen (Interlacing), hat sich als besonders nachteilig erwiesen. Die weitere computergestützte Bearbeitung und Auswertung der Bilder (z.B. die Bestimmung von Lage, Größe, Form etc. von Blutgefäßen im Auge 12) wird durch derartige Bewegungsartefakte oder Interlacing-Artefakte erschwert. Die erfindungsgemäße Vermeidung der mechanischen Schlitzblende vor dem Sensor löst all diese Probleme.

e) Der in Resonanz schwingende mechanische Oszillator zum Antrieb der Schlitzblenden beim Ophthalmoskop des Stands der Technik führt in der Regel eine sinusförmige Bewegung aus. Die integral während einer Periode durch die schwingende Blende auf das Auge 12 fallende Lichtmenge ist daher ungleichmäßig über das Auge 12 verteilt und führt zu einer ungleichmäßigen Kontrast- und Helligkeitsverteilung im Bild. Um dennoch eine gleichmäßige Ausleuchtung des Bildausschnitts zu erhalten, wird die Amplitude der Schwingung häufig so weit erhöht, dass nur der näherungsweise lineare Anteil der Blendentrajektorie im Sichtfeld liegt. Dadurch wird jedoch nur ein Bruchteil des auf das Auge 12 fallenden Lichts zur Bildgebung benutzt, was zu einer Verringerung der Sensitivität des Ophthalmoskops 10 führt. Beim erfindungsgemäßen Ophthalmoskop 10 hingegen wird das verfügbare Licht wesentlich besser ausgenutzt.

f) Durch die Verwendung einer herkömmlichen Glühlampe oder Halogenlampe, die die Blendenebene des Beleuchtungsstrahlenganges gleichmäßig ausleuchtet, wird die verfügbare Leuchtkraft der Beleuchtungsvorrichtung 14 nur mangelhaft ausgenutzt. Die Wärmeabstrahlung der Beleuchtungsvorrichtung 14 führt zu einer weiteren Beeinträchtigung der Handhabung des Gerätes. Diese Probleme lassen sich bei der in Figur 5 gezeigten Ausführungsform mit einem Laser und einer Linienfokussieroptik vermeiden.

g) Eine Halogenlampe, wie sie im Stand der Technik eingesetzt wird, strahlt ein breites, thermisches Frequenzspektrum ab, dessen Maximum im Infrarotbereich liegt, und das zu kürzeren Wellenlängen hin abfällt. Eine solche Lichtquelle ist dafür ungeeignet, ein kontrastreiches Farbbild des Augenhintergrunds zu erzeugen, da der Augenhintergrund hauptsächlich im roten Spektralbereich reflektiert. Halbleiterbasierte Bildsensoren sind zudem stärker im roten und infraroten Spektralbereich empfindlich als bei kürzeren Wellenlängen. Ein mit Halogenbeleuchtung aufgenommenes Farbbild des Augenhintergrunds hat daher einen dominanten Rot-Kanal, jedoch nur schwach ausgesteuerte Grün- und Blau-Kanäle. Das Bild ist daher verrauscht und kontrastarm. Der Einsatz von Filtern zur Beeinflussung des Beleuchtungspektrums verbessert zwar den Farbeindruck, reduziert jedoch stark die Leuchtkraft der Lichtquelle. Für die bereits angesprochene Beobachtung von Fluoreszenzemissionen ist aber eine intensive Anregung der Farbstoffe in einem engen Spektralbereich notwendig. Dies können in der Ausführungsform gemäß Figur 5 moderne Lichtquellen wie z.B. LEDs oder Laser leisten.

**Patentansprüche**

1. Ophthalmoskop (10) zur Untersuchung eines Auges (12) eines Patienten, umfassen:

   eine Beleuchtungsvorrichtung (14) zur Erzeugung eines Beleuchtungsstrahls (19), wobei die Beleuchtungsvorrichtung (14) eine Lichtquelle umfasst zur Erzeugung von Anregungslicht mit einer Anregungsfrequenz im Beleuchtungsstrahl zum Anregen von Fluoreszenzemissionen im Hintergrund des Auges (12); eine Beleuthtungs-Abbildungsoptik zum Abbilden des Beleuchtungsstrahls (19) auf das Auge (12), und Mittel (18) zum Scannen des Beleuchtungsstrahls (19) über das Auge (12), sowie eine Beobachtungsvorrichtung (30), die einen elektronischen ersten Sensor (34) mit einem Feld von fotosensitiven Pixeln (36) sowie einen zweiten Sensor (68) umfaßt; eine Beobachtungs-Abbildungsoptik zum Abbilden eines durch Reflexion des Beleuchtungsstrahls (19) am Auge (12) erzeugten Beobachtungsstrahls auf die Beobachtungsvorrichtung (30), und Mittel (32) zum Ausblenden von Streulicht aus dem Beobachtungsstrahl, **dadurch gekennzeichnet, dass** in einem Beobachtungsstrahlengang ein dichroitischer Strahlteiler (65) angeordnet ist zum Aufteilen von Licht, das vom Auge (12) ausgeht, in einen die Fluoreszenzemissionen beinhaltenden ersten Anteil und einen zweiten Anteil, der am Auge (12) reflektiertes Anregungslicht beinhaltet, wobei ferner der erste Anteil des Lichts auf den ersten Sensor (34) gerichtet ist zum Erzeugen einer ersten Bildfolge von Fluoreszenzbildern und der zweite Anteil des Lichts auf den zweiten Sensor (68) gerichtet ist zum Erzeugen einer zweiten Bildfolge von Einzelbildern, wobei ferner eine elektronische Kontrolleinheit (38) des Ophthalmoskops (10) dazu ausgelegt ist, geometrische Transformationen zwischen den Einzelbildern der zweiten Bildfolge zu berechnen, Inverse der geometrischen Transformationen auf synchron mit den Einzelbildern aufgenommene Fluoreszenzbilder anzuwenden und die Fluoreszenzbilder durch Addition zu mitteln.

2. Ophthalmoskop (10) aus Anspruch 1, **dadurch gekennzeichnet, dass** eine zusätzliche Lichtquelle (72) vorgesehen ist zur Beleuchtung des Auges mit zusätzlichem Licht, welches Frequenzen aufweist, die sich von der Anregungsfrequenz und von Frequenzen der Fluoreszenzemissionen unterscheiden, wobei der dichroitische Strahlteiler (65) ausgestaltet ist zum Aufteilen von Licht in den die Fluoreszenzemissionen beinhaltenden ersten Anteil und den zweiten Anteil, der außer dem am Auge (12) reflektierten Anregungslicht auch am Auge (12) reflektiertes Licht der zusätzlichen Lichtquelle (72) beinhaltet.

3. Ophthalmoskop (10) nach einem der vorhergehenden Ansprüche, **dadurch gekennzeichnet, dass** es einen vor dem ersten Sensor (34) angeordneten ersten Filter (67) und/oder einen vor dem zweiten Sensor (68) angeordneten zweiten Filter (69) umfaßt zur Verbesserung einer Filterwirkung des dichroitischen Strahlteilers (65).

4. Ophthalmoskop (10) nach einem der vorhergehenden Ansprüche, **dadurch gekennzeichnet, dass** die Beleuchtungsvorrichtung (14) einen Laser oder eine LED umfasst.

5. Ophthalmoskop (10) nach einem der vorhergehenden Ansprüche, **dadurch gekennzeichnet, dass** die fotosensitiven Pixel (36) des ersten Sensors (34) jeweils zeilenweise aktivierbar und/oder auslesbar sind und die Mittel (32) zum Ausblenden von Streulicht aus dem Beobachtungsstrahl eine elektronische Steuerschaltung zum Auslesen wenigstens einer Pixelzeile des ersten Sensors umfassen, wobei das Ophthalmoskop (10) eine elektronische Kontrolleinheit (38) umfaßt, die dazu ausgelegt ist, die Mittel (18) zum Scannen und die elektronische Steuerschaltung derart zu vontrollieren, dass das Scannen des Beleuchtungsstrahls (19) über das Auge (12) synchron mit der Änderung der aktuell auszulesenden Pixelzeile erfolgt.

6. Ophthalmoskop (10) nach Anspruch 5, **dadurch gekennzeichnet, dass** die elektronische Steuerschaltung dazu ausgelegt ist, jeweils eine einzelne Pixelzeile auszulesen.

7. Ophthalmoskop (10) nach Anspruch 6, **dadurch gekennzeichnet, dass** die elektronische Steuerschaltung dazu ausgelegt ist, jeweils nach Ablauf einer einstellbaren Zeitverzögerung ($\Delta t$) die aktuell auszulesende Pixelzeile zu ändern, insbesondere von einer Pixelzeile zu einer benachbarten Pixelzeile.

8. Ophthalmoskop (10) nach einem der Ansprüche 5 bis 7, **dadurch gekennzeichnet, dass** die elektronische Steuerschaltung ferner dazu ausgelegt ist, jede Pixelzeile während einer einstellbaren Belichtungszeit ($t_{INT}$) vor ihrem Auslesen zu aktivieren.

9. Ophthalmoskop (10) nach einem der Ansprüche 5 bis 8, **dadurch gekennzeichnet, dass** die elektronische Steuerschaltung dazu ausgelegt ist, den Auslesevorgang infolge eines externen Triggersignales zu starten.

10. Ophthalmoskop (10) nach einem der vorhergehenden Ansprüche, dadurch gekenntzeichnet, dass die Mittel (18) zum Scannen des Beleuchtungsstrahls (19) über das Auge (12) einen schwenkbar gelagerten Spiegel (44) mit einer Scanvorrichtung umfassen.

11. Ophthalmoskop (10) nach Anspruch 10, **dadurch gekennzeichnet, dass** es ferner eine feste Spaltblende zum Auskoppeln eines linienförmigen Beleuchtungsstrahls (19) umfaßt.

12. Ophthalmoskop (10) nach Anspruch 10, **dadurch gekennzeichnet, dass** es ferner eine Linienfokussieroptik (40, 42) zum Fokussieren des Beleuchtungsstrahls (19) in einer Linie umfaßt.

13. Ophthalmoskop (10) nach Anspruch 12, **dadurch gekennzeichnet, dass** die Linienfokussieroptik (40, 42) eine Zylinderlinse (42) umfaßt.

14. Ophthalmoskop (10) nach einem der Ansprüche 10 bis 13, **dadurch gekennzeichnet, dass** die elektronische Kontrolleinheit (38) dazu ausgelegt ist, die elektronische Steuerschaltung des elektronischen ersten Sensors (34) und die Scanvorrichtung des schwenkbaren Spiegels (44) zu kontrollieren.

**Claims**

1. Ophthalmoscope (10) for examining a patient's eye (12), comprising:

an illumination device (14) for generating an illumination beam (19)wherein the illumination device (14)comprises a light source for the generation of excitation light with an excitation frequency in the illumination beam for exciting fluorescent emissions in the background of the eye (12);
illumination imaging optics for imaging the illumination beam (19) onto the eye (12), and means (18) for scanning the illumination beam (19) over the eye (12), as well as
an observation device (30) which comprises a first electronic sensor (34) with an array of photosensitive pixels (36) as well as a second sensor (68);
observation imaging optics for imaging an observation beam generated by reflection of the illumination beam (19) from the eye (12) onto the observation device (30), and
means (32) for excluding stray light from the observation beam,
**characterised in that**
a dichroic beam splitter (65) is arranged in an observation beam path for splitting the light coming from the eye (12) into a first portion comprising the fluorescent emissions and into a second portion which comprises excitation light reflected at the eye (12), and wherein in addition the first portion of the light is directed to the first sensor (34) for generating a first series of fluorescent images and the second portion of the light being directed to the second sensor (68) for generating a second series of individual images, and wherein furthermore an electronic control unit (38) of the ophthalmoscope (10) is configured to calculate geometric tranformations between the individual images, to apply inverses of the geometric transformations to fluorescent images captured synchronously with the individual images and to average the fluorescent images by addition.

2. Ophthalmoscope (10) according to claim 1, **characterised in that** an additional light source (72) for illuminating the eye with additional light is provided, this additional light having frequencies which are different from the excitation frequency and frequencies of the fluorescent emissions, wherein the dichroic beam splitter (65) is configured to split the light into the first portion containing the fluorescent emissions and into the second portion which does not only contain the excitation light reflected at the eye (12) but also the light of the additional light source (72) reflected at the eye (12).

3. Ophthalmoscope (10) according to one of the preceding claims, **characterised in that** it comprises a first filter (67) arranged in front of the first sensor (34) and/or a second filter (69) arranged in front of the second sensor (68) for improving a filtering effect of the dichroic beam splitter (65).

4. Ophthalmoscope (10) according to one of the preceding claims, **characterised in that** the illumination device (14) comprises a laser or a LED.

5. Ophthalmoscope (10) according to one of the preceding claims, **characterised in that** the photosensitive pixel (36) of the first sensor (34) can be activated and/or read out in rows and that the means (32) for excluding stray light from the observation beam comprise an electronic control circuit for reading out at least one pixel row of the first sensor, wherein the ophthalmoscope (10) comprises an electronic control unit (38) which is adapted to control the scanning means (18) and the electronic control circuit so that the scanning of the illumination beam (19) over the eye (12) is synchronous with the change of the pixel row to be currently read out.

6. Ophthalmoscope (10) according to one of the preceding claims, **characterised in that** the electronic control circuit is adapted to read out a single pixel row, respectively.

7. Ophthalmoscope (10) according to Claim 6, **characterised in that** the electronic control circuit is adapted to change the pixel row to be currently read out, in particular from one pixel row to a neighbouring pixel row, respectively after an adjustable time delay ($\Delta$t) has elapsed.

8. Ophthalmoscope (10) according to one of claims 5 to 7, **characterised in that** the electronic control circuit is furthermore adapted to activate each pixel row for an adjustable exposure time ($t_{INT}$) before it is read out.

9. Ophthalmoscope (10) according to one of claims 5 to 8, **characterised in that** electronic control circuit is adapted to start the readout process in response to an external trigger signal.

10. Ophthalmoscope (10) according to one of the preceding claims, **characterised in that** the means (18) for scanning the illumination beam (19) over the eye (12) comprise a tiltably mounted mirror (44) with a scanning device.

11. Ophthalmoscope (10) according to claim 10, **characterised in that** it furthermore comprises a fixed slit shutter for extracting a linear illumination beam (19).

12. Ophthalmoscope (10) according to claim 10, **characterised in that** it furthermore comprises line focusing optics (40, 42) for focusing the illumination beam (19) in a line.

13. Ophthalmoscope (10) according to claim 12, **characterised in that** the line focusing optics (40, 42) comprise a cylinder lens (42).

14. Ophthalmoscope (10) according to one of claims 10 to 13, **characterised in that** electronic control unit (38) is adapted to control the electronic control unit of the first electronic sensor (34) and the scanning device of the tiltable mirror (44).

## Revendications

1. Ophtalmoscope (10) pour l'examen d'un oeil (12) d'un patient, comprenant :

   un dispositif d'éclairage (14) pour la production d'un faisceau d'éclairage (19), dans lequel le dispositif d'éclairage (14) comprend une source lumineuse pour la production d'une lumière d'excitation avec une fréquence d'excitation dans le faisceau d'éclairage pour la stimulation d'émissions de fluorescence dans le fond de l'oeil (12) ;

une optique d'imagerie d'éclairage pour reproduire le faisceau d'éclairage (19) sur l'oeil (12), et des moyens (18) pour effectuer le balayage du faisceau d'éclairage (19) par-dessus l'oeil (12), ainsi qu'un dispositif d'observation (30), qui comprend un premier capteur électronique (34) avec un champ de pixels photosensibles (36) ainsi qu'un deuxième capteur (68) ;

une optique d'imagerie d'observation pour reproduire un faisceau d'observation généré par la réflexion du faisceau d'éclairage (19) par l'oeil (12) sur le dispositif d'observation (30), et des moyens (32) pour le masquage de la lumière diffusée provenant du faisceau d'observation, **caractérisé en ce**

**qu'**un séparateur de faisceau (65) dichroïque est disposé dans un trajet optique d'observation pour diviser la lumière qui part de l'oeil (12) en une première partie comprenant les émissions de fluorescence et une deuxième partie qui comprend la lumière d'excitation réfléchie par l'oeil (12), en outre, la première partie de la lumière étant orientée sur un premier capteur (34) pour la production d'une première séquence d'images d'images de fluorescence et la deuxième partie de la lumière étant orientée sur le deuxième capteur (68) pour la création d'une deuxième séquence d'images d'images individuelles, en ce qu' en outre, une unité de contrôle électronique (38) de l'ophtalmoscope (10) est conçue pour calculer des transformations géométriques entre les images individuelles de la deuxième séquence d'images, pour employer les inverses des transformations géométriques sur des images de fluorescence prises de manière synchrone avec les images individuelles et pour moyenner les images de fluorescence par addition.

2. Ophtalmoscope (10) selon la revendication 1, **caractérisé en ce qu'**une source lumineuse supplémentaire (72) est prévue pour l'éclairage de l'oeil par de la lumière supplémentaire, laquelle présente des fréquences qui se différencient de la fréquence d'excitation et des fréquences des émissions de fluorescence, le séparateur de faisceau dichroïque (65) étant conçu pour la division de la lumière en la première partie comprenant les émissions de fluorescence et en la deuxième partie qui comprend, en plus de la lumière d'excitation réfléchie par l'oeil (12), également la lumière de la source lumineuse supplémentaire (72) réfléchie par l'oeil (12).

3. Ophtalmoscope (10) selon l'une des revendications précédentes, **caractérisé en ce qu'**il comprend un premier filtre (67) disposé devant le premier capteur (34) et/ou un deuxième filtre (69) disposé devant le deuxième capteur (68) pour une amélioration de l'effet filtrant du séparateur de faisceau dichroïque (65).

4. Ophtalmoscope (10) selon l'une des revendications précédentes, **caractérisé en ce que** le dispositif d'éclairage (14) comprend un laser ou une diode électroluminescente.

5. Ophtalmoscope (10) selon l'une des revendications précédentes, **caractérisé en ce que** les pixels photosensibles (36) du premier capteur (34) peuvent être chacun activés et/ou peuvent être lus ligne par ligne et que les moyens (32) pour masquer la lumière diffusée provenant du faisceau d'observation comprennent un circuit de commande électronique pour la lecture d'au moins une ligne de pixels du premier capteur, l'ophtalmoscope (10) comprenant une unité de contrôle électronique (38) qui est conçue pour contrôler les moyens (18) pour effectuer le balayage et le circuit de commande électronique de telle sorte que le balayage du faisceau d'éclairage (19) par-dessus l'oeil (12) est effectué de manière synchrone avec la modification de la ligne de pixels à lire à cet instant.

6. Ophtalmoscope (10) selon la revendication 5, **caractérisé en ce que** le circuit de commande électronique est conçu pour lire chaque fois une ligne de pixels individuelle.

7. Ophtalmoscope (10) selon la revendication 6, **caractérisé en ce que** le circuit de commande électronique est conçu pour modifier chaque fois la ligne de pixels à lire à cet instant après l'écoulement d'un délai en temps ($\Delta$t) réglable, en particulier, en passant d'une ligne de pixels à une ligne de pixels voisine.

8. Ophtalmoscope (10) selon l'une des revendications 5 à 7, **caractérisé en ce que** le circuit de commande électronique est en outre conçu pour activer chaque ligne de pixels pendant une durée d'exposition ($t_{INT}$) réglable avant sa lecture.

9. Ophtalmoscope (10) selon l'une des revendications 5 à 8, **caractérisé en ce que** le circuit de commande de commutateur électronique est conçu pour démarrer le processus de la lecture à la suite d'un signal de déclenchement extérieur.

10. Ophtalmoscope (10) selon l'une des revendications précédentes, **caractérisé en ce que** les moyens (18) pour effectuer le balayage du faisceau d'éclairage (19) par-dessus l'oeil (12) comprennent un miroir (44) monté oscillant avec un dispositif de balayage.

11. Ophtalmoscope (10) selon la revendication 10, **ca-**

**ractérisé en ce qu'**il comprend en outre un diaphragme à fente fixe pour le découplage d'un faisceau d'éclairage (19) en forme de lignes.

12. Ophtalmoscope (10) selon la revendication 10, **caractérisé en ce qu'**il comprend en outre une optique de focalisation de lignes (40, 42) pour la focalisation du faisceau d'éclairage (19) suivant une ligne.

13. Ophtalmoscope (10) selon la revendication 12, **caractérisé en ce que** l'optique de focalisation de lignes (40, 42) comprend une lentille cylindrique (42).

14. Ophtalmoscope (14) selon l'une des revendications 10 à 13, **caractérisé en ce que** l'unité de contrôle électronique (38) est conçue pour contrôler le circuit de commande électronique du premier capteur électronique (34) et le dispositif de balayage du miroir (44) oscillant.

**Fig. 1 (Stand der Technik)**

**Fig. 2**

Zeitpunkt: $t_1$

Zeile: 1 — Auslesen
2
3 ~36
4
5
6
7
8 ↕ Shutter-Breite
9
10

**Fig. 3a**

Zeitpunkt: $t_2$

Zeile: 1 — Auslesen
2
3
4
5
6
7 ~36
8
9
10

**Fig. 3b**

Zeitpunkt: $t_{10}$

36

Zeile: 1
2
3
4
5
6
7
8
9
10 — Auslesen

**Fig. 3c**

Fig. 4a

Fig. 4b

EP 2 040 607 B1

Fig. 5

**Fig. 6**

**Fig. 7**

Fig. 8

Fig. 9

**Fig. 10**

**Fig. 11**

**Fig. 12**

**Fig. 13**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1389943 B1
- WO 9905853 A
- US 6758564 B2